# EUROPEAN PATENT SPECIFICATION

(11) **EP 3 114 276 B1**
(45) Date of publication and mention of the grant of the patent: **16.01.2019**
(21) Application number: 15710691.5
(22) Date of filing: 05.03.2015
(51) Int. Cl.: D21H 17/00, C12N 9/10, D06M 16/00

(54) **COMPOSITIONS AND METHODS FOR FUNCTIONALIZING AND LINKING MATERIALS**
ZUSAMMENSETZUNGEN UND VERFAHREN ZUR FUNKTIONALISIERUNG UND VERBINDUNG VON MATERIALIEN
COMPOSITIONS ET MÉTHODES DE FONCTIONNALISATION ET DE LIAISON DE MATÉRIAUX

(30) Priority: 05.03.2014 US 201461948229 P
(43) Date of publication of application: 11.01.2017
(73) Proprietor: Novozymes A/S, 2880 Bagsvaerd (DK)
(72) Inventor: BERLIN, Alex, Davis, California 95616 (US); QUINLAN, Jason, Woodland, California 95695 (US); BENYAMINO, Romil, Sacramento, California 95818 (US)
(74) Representative: NZ EPO Representatives
(86) International application number: PCT/US2015/019008
(87) International publication number: WO 2015/134773

(56) References cited:
- WO-A1-97/23683
- WO-A1-2004/094646
- WO-A1-2006/079512
- WO-A1-2007/147428
- R Okasawa: "Full=Xyloglucan endotransglucosylase/hydrolase protein A; Alt - Protein - NCBI", , 19 February 2014 (2014-02-19), XP055188280, Retrieved from the Internet: URL:http://www.ncbi.nlm.nih.gov/protein/38 605156?sat=18&satkey=20413929 [retrieved on 2015-05-08] -& NAKAMURA T ET AL: "TWO AZUKI BEAN XTH GENES, VAXTH1 AND VAXTH2, WITH SIMILAR TISSUE-SPECIFIC EXPRESSION PROFILES, ARE DIFFERENTLY REGULATED BY AUXIN", PLANT AND CELL PHYSIOLOGY, OXFORD UNIVERSITY PRESS, UK, vol. 44, no. 1, 1 January 2003 (2003-01-01), pages 16-24, XP009038415, ISSN: 0032-0781, DOI: 10.1093/PCP/PCG002
- P Campbell: "Full=Xyloglucan endotransglucosylase/hydrolase protein 14; Sh - Protein - NCBI", , 22 February 2014 (2014-02-22), XP055188302, Retrieved from the Internet: URL:http://www.ncbi.nlm.nih.gov/protein/38 605534?sat=18&satkey=5934425 [retrieved on 2015-05-08] -& PAUL CAMPBELL ET AL: "In vitro activities of four xyloglucan endotransglycosylases from Arabidopsis", THE PLANT JOURNAL, vol. 18, no. 4, 1 May 1999 (1999-05-01), pages 371-382, XP55024888, ISSN: 0960-7412, DOI: 10.1046/j.1365-313X.1999.00459.x

## Description

### Reference to a Sequence Listing

This application contains a Sequence Listing in computer readable form.

### Background of the Invention

### Field of the Invention

The present invention relates to compositions and methods for functionalizing and linking materials.

### Description of the Related Art

Xyloglucan endotransglycosylase (XET) is an enzyme that catalyzes endotransglycosylation of xyloglucan, a structural polysaccharide of plant cell walls. The enzyme is present in most plants, and in particular, land plants. XET has been extracted from dicotyledons and monocotyledons.

Xyloglucan is present in cotton, paper, or wood fibers (Hayashi et al., 1988, Carbohydrate Research 181: 273-277) making strong hydrogen bonds to cellulose (Carpita and Gibeaut, 1993, The Plant Journal 3: 1-30). Adding xyloglucan endotransglycosylase to various cellulosic materials containing xyloglucan alters the xyloglucan mediated interlinkages between cellulosic fibers improving their strength, and maintaining the cellulose-structure while permitting the cellulose fibers to move relative to one another under force.

WO 97/23683 discloses a process for providing a cellulosic material, such as a fabric or a paper and pulp product, with improved strength and/or shape-retention and/or anti-wrinkling properties by using xyloglucan endotransglycosylase. WO 2001/07556 discloses laundry and/or fabric and/or color care compositions containing xyloglucan endotransglycosylase in combination with a polysaccharide and/or oligosaccharide to refurbish and/or restore improved tensile strength, enhanced anti-wrinkle, anti-bobbling and anti-shrinkage properties to fabrics.

Functionalized and surface-functionalized materials are desired for use in a wide variety of applications, such as building materials, textiles, plastics, food products, or packaging. There is a need in the art to develop inexpensive, robust and/or flexible materials with surfaces that have an affinity or chemical reactivity towards various chemical, radiological or biological compounds, which can be used for diagnostics, disposal, cleaning, sequestration or bioremediation of the compounds.

There is a need in the art to develop composite materials by blending natural with synthetic polymers; to generate methods for improving association between synthetic and natural polymers either via the polymers themselves or via functionalized fillers that have improved association properties; to link minerals or metals with polymers; and to impart electrical conductivities, magnetic properties, optical properties, physical properties, mechanical properties, or chemical properties to flexible, thin, or easily shaped materials.

The present invention provides compositions and methods for functionalizing and linking materials.

### Summary of the Invention

The present invention relates to methods for
linking materials comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; and (h) a composition comprising a xyloglucan endotransglycosylase and a xyloglucan oligomer, under conditions leading to a modified material comprising polymeric xyloglucan functionalized with a chemical group, under conditions leading to linking between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan:xyloglucan xyloglucanotransferase (EC 2.4.1.207).

The present invention also relates to methods for linking materials comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, and (i) a composition of (a), (b), (c), (d), (e), (f), (g), or (h) without a xyloglucan endotransglycosylase, under conditions leading to the formation of a linkage between the two or more materials.

The present invention also relates to methods for producing
a composite material comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; and (h) a composition comprising a xyloglucan endotransglycosylase and a xyloglucan oligomer, under conditions leading to a modified material comprising polymeric xyloglucan functionalized with a chemical group, under conditions leading to association between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan :xyloglucan xyloglucanotransferase (EC 2.4.1.207).

The present invention also discloses functionalized, linked, or composite materials obtained by such methods.

### Brief Description of the Figures

Figure 1 shows a restriction map of pDLHD0012.
Figure 2 shows a restriction map of pMMar27.
Figure 3 shows a restriction map of pEvFz1.
Figure 4 shows a restriction map of pDLHD0006.
Figure 5 shows a restriction map of pDLHD0039.
Figure 6 shows the effect of *Vigna angularis* xyloglucan endotransglycosylase 16 (VaXET16) modified kaolin on filler retention in handsheet compositions.
Figure 7 shows the increase of fluorescein isothiocyanate-labeled xyloglucan (FITC-XG) fluorescence associated with the solid phase after incubation with increasing masses of kaolin, relative to a control incubation performed without kaolin. Figure 7A shows kaolin titration after 1 day of incubation; Figure 7B shows kaolin titration after 2 days of incubation; and Figure 7C shows kaolin titration after 5 days of incubation.
Figure 8 shows fluorescence spectra of supernatants of various kaolin preparations. Figure 8A shows the fluorescence spectra of supernatants of various kaolin concentrations incubated without FITC-XG. Figure 8B shows the fluorescence spectra of supernatants of various kaolin concentrations incubated with FITC-XG. Figure 8C shows the fluorescence spectra of supernatants of various concentrations of kaolin incubated with FITC-XG and VaXET16.
Figure 9 shows FITC-XG bound to kaolin by confocal microscopy. Figure 9A shows the confocal microscopy image of kaolin incubated with no FITC-XG. Figure 9B shows the confocal microscopy image of kaolin incubated with FITC-XG. Figure 9C shows the confocal microscopy image of kaolin incubated with FITC-XG and VaXET16. The panels are overlays of transmission and fluorescence emission images.

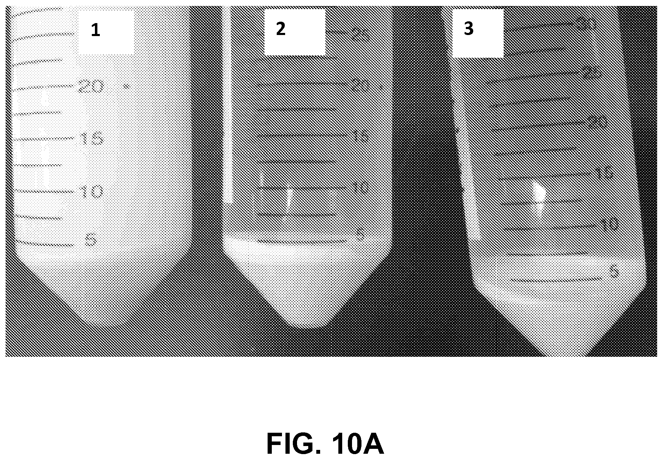

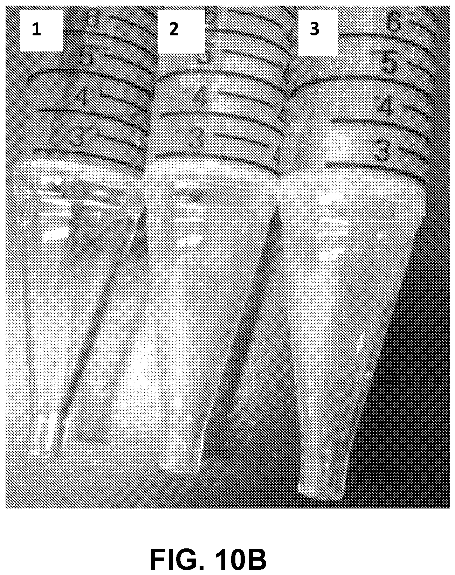

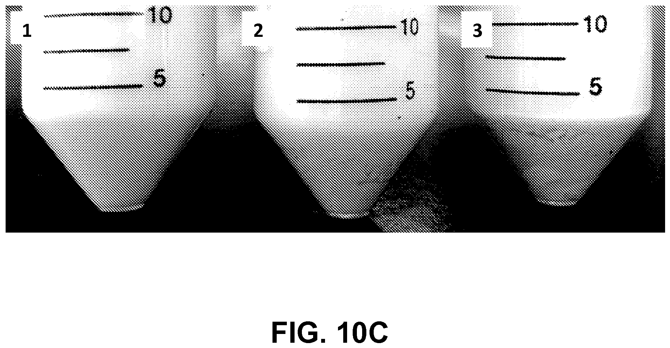

Figure 10 shows the effect on physical properties of kaolin after incubation with xyloglucan or xyloglucan and VaXET16. Figure 10A shows photographs of 50 ml conical tubes containing (1) kaolin, (2) kaolin incubated with xyloglucan, and (3) kaolin incubated with xyloglucan and VaXET16, following centrifugation. Figure 10B shows photographs of polystyrene serological pipets following contact with (1) kaolin, (2) kaolin incubated with xyloglucan, and (3) kaolin incubated with xyloglucan and VaXET16. Figure 10C shows photographs of 50 ml conical tubes containing (1) kaolin, (2) kaolin incubated with xyloglucan, and (3) kaolin incubated with xyloglucan and VaXET16, following extensive washing and resuspension in water.
Figure 11 shows the fluorescence intensity of the supernatants of FITC-XG TiO₂-binding reactions and control incubations at various times. Open circles: TiO₂ with no FITC-XG; squares: TiO₂ with FITC-XG; diamonds: TiO₂ with FITC-XG and *Arabidopsis thaliana* xyloglucan endotransglycosylase 14 (AtXET14); triangles: FITC-XG with no TiO₂.
Figure 12 shows the fluorescence image of various test fabrics incubated with FITC-labeled silica and VaXET16, xyloglucan, xyloglucan oligomers or combinations as indicated.
Figure 13 shows the mean fluorescence intensities of various test fabrics incubated with FITC-labeled silica and VaXET16, xyloglucan, xyloglucan oligomers or combinations as indicated.

### Definitions

As used herein, the singular forms "a", "an", and "the" are intended to include the plural forms as well, unless the context clearly indicates otherwise.

**Composite material:** The term "composite material" means a material that has been generated by combining 2 or more constituent materials in a manner wherein each constituent material maintains the properties of that material and the final composite material has properties that differ from those of the constituent materials. Examples of composite materials are concrete, metal (rebar)-reinforced concrete, wood plastic composite building materials, wood composites, glass or carbon fiber reinforced plastic, particle board, and metal ceramic composites.

**Functionalized xyloglucan oligomer:** The term "functionalized xyloglucan oligomer" means a short chain xyloglucan oligosaccharide, including single or multiple repeating units of xyloglucan, which has been modified by incorporating a chemical group. The xyloglucan oligomer is preferably 1 to 3 kDa in molecular weight, corresponding to 1 to 3 repeating xyloglucan units. The chemical group may be a compound of interest or a reactive group such as an aldehyde group, an amino group, an aromatic group, a carboxyl group, a halogen group, a hydroxyl group, a ketone group, a nitrile group, a nitro group, a sulfhydryl group, or a sulfonate group. The incorporated reactive groups can be derivatized with a compound of interest or can be used to coordinate metal cations and/or to bind other chemical entities that interact (*e.g.,* covalently, hydrophobically, electrostatically, etc.) with the reactive groups. The derivatization can be performed directly on a functionalized xyloglucan oligomer comprising a reactive group or after the functionalized xyloglucan oligomer comprising a reactive group is incorporated into polymeric xyloglucan. Alternatively, the xyloglucan oligomer can be functionalized by incorporating directly a compound by using a reactive group contained in the compound, *e.g.,* an aldehyde group, an amino group, an aromatic group, a carboxyl group, a halogen group, a hydroxyl group, a ketone group, a nitrile group, a nitro group, a sulfhydryl group, or a sulfonate group. The terms "functionalized xyloglucan oligomer" and "functionalized xyloglucan oligomer comprising a chemical group" are used interchangedly herein.

**Linked material:** The term "linked material" means two or more materials that are connected through, cross-linked by, or coated over with xyloglucan in a manner that connects or bridges the materials together. In an embodiment, the linkage between the materials occurs via xyloglucan or xyloglucan derivatives forming covalent or non-covalent associations with the two or more materials. In another embodiment, the linkage between the materials occurs via xyloglucan, covalently linked to a chemical group or material, forming covalent or non-covalent associations with one or more materials. In another embodiment, the linked material is entrapped or enmeshed by xyloglucan associations between two or more molecules of the same material. In another embodiment, the linkages between the materials are optimized, strengthened or increased in number by the activity of a xyloglucan endotransglycosylase.

**Polymeric xyloglucan:** The term "polymeric xyloglucan" means short, intermediate or long chain xyloglucan oligosaccharide or polysaccharide encompassing more than one repeating unit of xyloglucan, *e.g.,* multiple repeating units of xyloglucan. Most optimally, polymeric xyloglucan encompasses xyloglucan of 50-200 kDa number average molecular weight, corresponding to 50-200 repeating units. A repeating motif of xyloglucan is composed of a backbone of four beta-(1-4)-D-glucopyranose residues, three of which have a single alpha-D-xylopyranose residue attached at O-6. Some of the xylose residues are beta-D-galactopyranosylated at O-2, and some of the galactose residues are alpha-L-fucopyranosylated at O-2. The term "xyloglucan" herein is understood to mean polymeric xyloglucan.

**Polymeric xyloglucan functionalized with a chemical group:** The term "polymeric xyloglucan functionalized with a chemical group" means a polymeric xyloglucan that has been modified by incorporating a chemical group. The polymeric xyloglucan is short, intermediate or long chain xyloglucan oligosaccharide or polysaccharide encompassing more than one repeating unit of xyloglucan, *e.g.,* multiple repeating units of xyloglucan. The polymeric xyloglucan encompasses xyloglucan of 50-200 kDa number average molecular weight, corresponding to 50-200 repeating units. A repeating motif of xyloglucan is composed of a backbone of four beta-(1-4)-D-glucopyranose residues, three of which have a single alpha-D-xylopyranose residue attached at 0-6. The chemical group may be a compound of interest or a reactive group such as an aldehyde group, an amino group, an aromatic group, a carboxyl group, a halogen group, a hydroxyl group, a ketone group, a nitrile group, a nitro group, a sulfhydryl group, or a sulfonate group. The chemical group can be incorporated into a polymeric xylogucan by reacting the polymeric xyloglucan with a functionalized xyloglucan oligomer in the presence of xyloglucan endotransglycosylase. The incorporated reactive groups can then be derivatized with a compound of interest. The derivatization can be performed directly on a functionalized polymeric xyloglucan comprising a reactive group or after a functionalized xyloglucan oligomer comprising a reactive group is incorporated into a polymeric xyloglucan. Alternatively, the polymeric xyloglucan can be functionalized by incorporating directly a compound by using a reactive group contained in the compound, *e.g.,* an aldehyde group, an amino group, an aromatic group, a carboxyl group, a halogen group, a hydroxyl group, a ketone group, a nitrile group, a nitro group, a sulfhydryl group, or a sulfonate group.

**Xyloglucan endotransglycosylase:** The term "xyloglucan endotransglycosylase" means a xyloglucan:xyloglucan xyloglucanotransferase (EC 2.4.1.207) that catalyzes cleavage of a β-(1→4) bond in the backbone of a xyloglucan and transfers the xyloglucanyl segment on to 0-4 of the non-reducing terminal glucose residue of an acceptor, which can be a xyloglucan or an oligosaccharide of xyloglucan. Xyloglucan endotransglycosylases are also known as xyloglucan endotransglycosylase/hydrolases or endo-xyloglucan transferases. Some xylan endotransglycosylases can possess different activities including xyloglucan and mannan endotransglycosylase activities. For example, xylan endotransglycosylase from ripe papaya fruit can use heteroxylans, such as wheat arabinoxylan, birchwood glucuronoxylan, and others as donor molecules. These xylans can potentially play a similar role as xyloglucan while being much cheaper in cost since they can be extracted, for example, from pulp mill spent liquors and/or future biomass biorefineries.

Xyloglucan endotransglycosylase activity can be assayed by those skilled in the art using any of the following methods. The reduction in the average molecular weight of a xyloglucan polymer when incubated with a molar excess of xyloglucan oligomer in the presence of xyloglucan endotransglycosylase can be determined via liquid chromatography (Sulova et al., 2003, Plant Physiol. Biochem. 41: 431-437) or via ethanol precipitation (Yaanaka et al., 2000, Food Hydrocolloids 14: 125-128) followed by gravimetric or cellulose-binding analysis (Fry et al., 1992, Biochem. J. 282: 821-828), or can be assessed colorimetrically by association with iodine under alkaline conditions (Sulova et al., 1995, Analytical Biochemistry 229: 80-85). Incorporation of a functionalized xyloglucan oligomer into a xyloglucan polymer by incubation of the functionalized oligomer with xyloglucan in the presence of xyloglucan endotransglycosylase can be assessed, *e.g.,* by incubating a radiolabeled xyloglucan oligomer with xyloglucan and xyloglucan endotransglycosylase, followed by filter paper-binding and measurement of filter paper radioactivity, or incorporation of a fluorescently or optically functionalized xyloglucan oligomer can be assessed similarly, monitoring fluorescence or colorimetrically analyzing the filter paper.

**Xyloglucan oligomer:** The term "xyloglucan oligomer" means a short chain xyloglucan oligosaccharide, including single or multiple repeating units of xyloglucan. Most optimally, the xyloglucan oligomer will be 1 to 3 kDa in molecular weight, corresponding to 1 to 3 repeating xyloglucan units.

### Detailed Description of the Invention

The present invention discloses methods for functionalizing a material comprising treating the material with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, and (i) a composition of (a), (b), (c), (d), (e), (f), (g), or (h) without a xyloglucan endotransglycosylase, under conditions leading to a modified material comprising polymeric xyloglucan functionalized with a chemical group, wherein the modified material possesses an improved property compared to the unmodified material.

The present invention relates to methods for linking materials comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, and (i) a composition of (a), (b), (c), (d), (e), (f), (g), or (h) without a xyloglucan endotransglycosylase, under conditions leading to the formation of a linkage between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan:xyloglucan xyloglucanotransferase (EC 2.4.1.207).

The present invention also relates to methods for producing a composite material comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, or (i) a composition of (a), (b), (c), (d), (e), (f), (g), or (h) without a xyloglucan endotransglycosylase, under conditions leading to association between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan:xyloglucan xyloglucanotransferase (EC 2.4.1.207).

The disclosure also relates to functionalized, linked, or composite materials obtained by such methods.

The disclosure also relates to functionalized, linked, or composite materials comprising (a) a polymeric xyloglucan and a functionalized xyloglucan oligomer comprising a chemical group; (b) a polymeric xyloglucan functionalized with a chemical group and a functionalized xyloglucan oligomer comprising a chemical group; (c) a polymeric xyloglucan functionalized with a chemical group and a xyloglucan oligomer; (d) a polymeric xyloglucan and a xyloglucan oligomer; (e) a polymeric xyloglucan functionalized with a chemical group; (f) a polymeric xyloglucan; (g) a functionalized xyloglucan oligomer comprising a chemical group; or (h) a xyloglucan oligomer.

The disclosure further relates to a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, and (i) a composition of (a), (b), (c), (d), (e), (f), (g), or (h) without a xyloglucan endotransglycosylase.

In one embodiment, the composition comprises a xyloglucan endotransglycosylase, a polymeric xyloglucan, and a functionalized xyloglucan oligomer comprising a chemical group. In another embodiment, the composition comprises a xyloglucan endotransglycosylase, a polymeric xyloglucan functionalized with a chemical group, and a functionalized xyloglucan oligomer comprising a chemical group. In another embodiment, the composition comprises a xyloglucan endotransglycosylase, a polymeric xyloglucan functionalized with a chemical group, and a xyloglucan oligomer. In another embodiment, the composition comprises a xyloglucan endotransglycosylase, a polymeric xyloglucan, and a xyloglucan oligomer. In another embodiment, the composition comprises a xyloglucan endotransglycosylase and a polymeric xyloglucan functionalized with a chemical group. In another embodiment, the composition comprises a xyloglucan endotransglycosylase and a polymeric xyloglucan. In another embodiment, the composition comprises a xyloglucan endotransglycosylase and a functionalized xyloglucan oligomer comprising a chemical group. In another embodiment, the composition comprises a xyloglucan endotransglycosylase and a xyloglucan oligomer.

In another embodiment, the composition comprises a polymeric xyloglucan and a functionalized xyloglucan oligomer comprising a chemical group. In another embodiment, the composition comprises a polymeric xyloglucan functionalized with a chemical group and a functionalized xyloglucan oligomer comprising a chemical group. In another embodiment, the composition comprises a polymeric xyloglucan functionalized with a chemical group and a xyloglucan oligomer. In another embodiment, the composition comprises a polymeric xyloglucan and a xyloglucan oligomer. In another embodiment, the composition comprises a polymeric xyloglucan functionalized with a chemical group. In another embodiment, the composition comprises a polymeric xyloglucan. In another embodiment, the composition comprises a functionalized xyloglucan oligomer comprising a chemical group. In another embodiment, the composition comprises a xyloglucan oligomer.

It is well known in the art that functionalized materials and surface-functionalized materials are highly desired for use in a broad range of industries and for a broad range of applications. As an example, anti-microbial, anti-fungal, and/or anti-insect (insecticidal) functionalization of lumber or wood, building materials, textiles, plastics, food products, or packaging, etc., is highly desirable. In one particularly relevant example, it is highly desirable in the art to develop inexpensive, robust and/or flexible surfaces with affinity or chemical reactivity towards various chemical, radiological or biological compounds for use in diagnostics, disposal, cleaning, sequestration, or bioremediation of the compounds (*e.g.,* as diagnostic or cleaning wipes, or in larger scale for industrial or environmental cleanup, wherein the functionalized material can chemically neutralize the specific compounds or adsorb these specifically, or as resistant, self-cleaning or neutralizing surfaces).

It is also well known in the art that there is a need to link minerals with polymers. The polymers can be synthetic (*e.g.,* polystyrene, polyvinylchloride, polytetrafluoroethylene, polyethylene, polyethylene terephthalate, etc.) or natural (*e.g.,* cellulose, cellulose-derivatives, chitin, silk, carbon fiber). Minerals can be effective anti-microbial agents, imparting anti-microbial characteristics to a polymer composite, (*e.g.,* in the production of anti-microbial fabrics, etc.). Minerals can also impart resistance to ultraviolet light, thus providing a polymer with resistance to sun damage (*e.g.,* paints, polyurethanes, sealants, varnishes, resins, vinyl siding, storage or vinyl outdoor toys, etc.). There is also a desire in the art to impart specific electrical conductivities, magnetic properties, or specific optical properties to flexible, thin or easily shaped materials (*e.g.,* optically active fabrics, flexible television, smartphone and computer monitors, etc.). Minerals are associated with or are sandwiched between plastic polymers in the synthesis of LED, OLED and touch-sensitive screens, etc. Thus, there is need in the art for functionalization of the plastic surfaces with minerals to enhance or improve the manufacturing processes of these items.

It is also well known in the art that there is need to develop composite materials by, for example, blending natural polymers with synthetic polymers. Examples include building materials (*e.g.,* composite lumber or deck materials), and fabrics and textiles blends (*e.g.,* cotton-rayon, poly-cotton, etc.). Enhanced association or linkage between these component materials could enhance the tensile strength or other physical properties, allow more cost-effective blend ratios, and improve the manufacturing processes or reduce the costs of the manufacturing process (*e.g.,* by eliminating or reducing the need for blending or linking reagents and chemicals). There is also need to generate methods for improving association between synthetic and natural polymers either via the polymers themselves or via functionalized fillers that have improved association properties (*e.g.,* latex or water-based paint associations with wood or with oil-based paint). Within textile and apparel manufacturing, textiles comprising synthetic polymers (*e.g.,* nylon, polyester, etc.) are known to feel unnatural, and there is need in the art to generate a more natural or cotton-like feel, and more cotton-like performance in, for example, synthetic textile athletic apparel. Enhanced blending of, for example, polyester and cotton, or a natural polymer such as xyloglucan could be used to generate the desired performance and feel improvements.

In one aspect, the functionalization can provide any functionally useful chemical moiety. In another aspect, the linkage can provide any functionally useful composite.

The functionalization of a material can be performed in any useful medium. In an embodiment, the medium is an aqueous medium. In another embodiment, the medium is a partially aqueous medium. In another aspect, the medium is a slurry. In another aspect, the medium is an aqueous slurry. In another aspect, the medium is a non-aqueous slurry. In another aspect, the medium is a partially aqueous slurry. In another aspect, the medium is a waxy suspension. In another aspect, the medium is an emulsion.

Additionally, the opposing activities of xyloglucanase, endo-β-1-4 glucanase, cellulase, or combinations thereof and xyloglucan endotransglycosylase can be exploited to permit the functionalization to be enzymatically removed and reintroduced as desired. The subsequently unfunctionalized material is refunctionalized by addition of polymeric xyloglucan functionalized with a chemical group or functionalized xyloglucan oligomers and xyloglucan endotransglycosylase.

The xyloglucan endotransglycosylase is preferably present at about 0.1 nM to about 1 mM, *e.g.,* about 10 nM to about 100 µM or about 0.5 µM to about 5 µM, in the composition.

The polymeric xyloglucan or polymeric xyloglucan functionalized with a chemical group is preferably present at about 10 mg to about 1 g per g of the composition, *e.g.,* about 100 mg to about 950 mg or about 500 mg to about 900 mg per g of the composition.

When the xyloglucan oligomer or the functionalized xyloglucan oligomer is present without polymeric xyloglucan or polymeric xyloglucan functionalized with a chemical group, the xyloglucan oligomer or the functionalized xyloglucan oligomer is preferably present at about 10 mg to about 1 g per g of the composition, *e.g.,* about 100 mg to about 950 mg or about 500 mg to about 900 mg per g of the composition.

When present with the polymeric xyloglucan or polymeric xyloglucan functionalized with a chemical group, the xyloglucan oligomer or the functionalized xyloglucan oligomer is preferably present with the polymeric xyloglucan at about 50:1 to about 0.5:1 molar ratio of xyloglucan oligomer or functionalized xyloglucan oligomer to polymeric xyloglucan or polymeric xyloglucan functionalized with a chemical group, *e.g.,* about 10:1 to about 1:1 or about 5:1 to about 1:1 molar ratio of xyloglucan oligomer or functionalized xyloglucan oligomer to polymeric xyloglucan or polymeric xyloglucan functionalized with a chemical group.

The polymeric xyloglucan or polymeric xyloglucan functionalized with a chemical group is preferably present at about 1 mg to about 1 g per g of the material, *e.g.,* about 10 mg to about 100 mg or about 20 mg to about 50 mg per g of the material.

When the xyloglucan oligomer or the functionalized xyloglucan oligomer is present without polymeric xyloglucan or polymeric xyloglucan functionalized with a chemical group, the xyloglucan oligomer or the functionalized xyloglucan oligomer is preferably present at about 1 mg to about 1 g per g of the material, *e.g.,* about 10 mg to about 100 mg or about 20 mg to about 50 mg per g of the material

When present with the polymeric xyloglucan or polymeric xyloglucan functionalized with a chemical group, the xyloglucan oligomer or the functionalized xyloglucan oligomer is preferably present at about 50:1 to about 0.5:1, *e.g.,* about 10:1 to about 1:1 or about 5:1 to about 1:1 molar ratio of xyloglucan oligomer or functionalized xyloglucan oligomer to polymeric xyloglucan or polymeric xyloglucan functionalized with a chemical group.

The xyloglucan endotransglycosylase is preferably present at about 0.1 nM to about 1 mM, *e.g.,* about 10 nM to about 100 µM or about 0.5 µM to about 5 µM.

The concentration of polymeric xyloglucan, polymeric xyloglucan functionalized with a chemical group, xyloglucan oligomer, or functionalized xyloglucan oligomer comprising a chemical group incorporated into the material is about 0.01 g to about 500 mg per g of material, *e.g.* about 0.1 g to about 50 mg or about 1 to about 5 mg per g of material.

### Materials

In the methods of the present invention, the material can be any material. The material can be a cellulosic material (*e.g.,* paper, board, pulp, cotton, wool, yarn, jute, cellulosic and lignocellulosic biomass). In an embodiment, the cellulosic material is comprised of purified or semi-purified cellulose (*e.g.,* filter paper, cotton). In another embodiment, the cellulosic material is comprised of unpurified, raw or enriched cellulose (*e.g.,* biomass, lignocellulose). In another embodiment, the cellulosic material is a derivative of cellulose (*e.g.,* cellulose acetate, carboxymethylcellulose, rayon, viscose, and nitrocellulose lacquer).

The material can also be a natural, non-cellulosic polymer (*e.g.,* amber, shellac).

The material can also be a synthetic polymer (*e.g.,* polystyrene, polyethylene, polyethylene terephthalate, polyvinylchloride, polypropylene, polyacrylonitrile, polytetrafluoroethylene, polyurethane, acrylic, latex, spandex, nylon, polyacrylamide, polyacrylic acid, polylactic acid, and silicone). In an embodiment, the synthetic polymer is formed into a fabric or textile. In another embodiment, the synthetic polymer is used as a plastic. In another embodiment, the synthetic polymer is a thermoset. In another embodiment, the synthetic polymer is a plasticizer.

The material can also be a homopolymer, a copolymer, or a heteropolymer (*e.g.,* ABS plastic, SBR, nitrile rubber, styrene-isoprene-styrene, styrene-butadiene, ethylene-vinyl acetate).

The material can also be a mineral (*e.g.,* kaolin, silica, titanium (IV) oxide, rutile, anatase, aluminum oxides, aluminum hydroxides, iron oxide, iron sulfide, magnetite, pyrite, hematite, ferrite, gregite, calcium carbonate, calcite, aragonite, quartz, zircon, olivine, orthopyroxene, tourmaline, kyanite, albite, anorthite, clinopyroxene, orthoclase, gypsum, andalusite, talc, fluorite, apatite, orthoclase, topaz, corundum, diamond, tin, tin oxides, antimony, antimony oxides, beryllium, cobalt, copper, feldspar, gallium, indium, lead, lithium, manganese, mica, molybdenum, nickel, perlite, platinum group metals, phosphorous and phosphate rock, potash, rare earth elements, tantalum, tungsten, vanadium, zeolites, zinc and zinc oxide, and indium tin oxide). In an embodiment, the minerals are used as mined or extracted. In another embodiment, the minerals are refined or purified in a manner familiar to those skilled in the art. In a preferred embodiment, the minerals are metal hydroxides or metal oxides, which provide a higher affinity association with xyloglucan orfunctionalized xyloglucan. In another embodiment, the metal lattice or crystal lattice of a mineral is doped to provide additional characteristics to the material.

### Improved Properties

Treatment of a material according to the methods of the present invention imparts an improved property to the material.

The improved property can be one or more improvements including, but are not limited to, rot resistance, decay resistance, senescence resistance, oxidation resistance, flame or chemical resistance, UV resistance, enhanced color properties, improved dye or color uptake or retention, specific affinities, specific chemical reactivity, fungicidal or fungistatic properties, herbicidal or herbistatic properties, bacteriocidal or bacteriostatic properties, microbiocidal or microbiostatic properties, non-specific affinities, chemical reactivity or affinity towards or resistance to specific chemicals, chemical groups, toxins, metals, salts, ions, polypeptides or desired analytes, including biological, radiological, chemical, etc., enhanced water-repellency, weather resistance, enhanced tensile strength, tear resistance, altered or improved optical properties, conductive properties, thermal properties, enzymatic activities, non-enzymatic catalytic activities, blending or association properties, anti-wrinkling, anti-piling, anti-shrink, surface properties, hydrophobicity, hydrophilicity, feel, texture, or hand feel properties, magnetic properties, rheology, and compositional properties.

In one aspect, the improved property is rot resistance. In another aspect, the improved property is decay resistance. In another aspect, the improved property is senescence resistance. In another aspect, the improved property is oxidation resistance. In another aspect, the improved property is flame resistance. In another aspect, the improved property is chemical resistance. In another aspect, the improved property is UV resistance. In another aspect, the improved property is enhanced color properties. In another aspect, the improved property is improved dye or color uptake or retention. In another aspect, the improved property is specific affinities. In another aspect, the improved property is specific chemical reactivity. In another aspect, the improved property is fungicidal or fungistatic properties. In another aspect, the improved property is herbicidal or herbistatic properties. In another aspect, the improved property is bacteriocidal or bacteriostatic properties. In another aspect, the improved property is microbiocidal or microbiostatic properties. In another aspect, the improved property is non-specific affinities. In another aspect, the improved property is chemical reactivity or affinity towards or resistance to specific chemicals, chemical groups, toxins, metals, salts, ions, polypeptides or desired analytes. In another aspect, the improved property is enhanced water-repellency. In another aspect, the improved property is weather resistance. In another aspect, the improved property is enhanced tensile strength. In another aspect, the improved property is tear resistance. In another aspect, the improved property is altered or improved optical properties. In another aspect, the improved property is conductive properties. In another aspect, the improved property is thermal properties. In another aspect, the improved property is enzymatic activities. In another aspect, the improved property is non-enzymatic catalytic activities. In another aspect, the improved property is blending or association properties. In another aspect, the improved property is anti-wrinkling properties. In another aspect, the improved property is anti-piling properties. In another aspect, the improved property is anti-shrink properties. In another aspect, the improved property is surface properties. In another aspect, the improved property is hydrophobicity. In another aspect, the improved property is hydrophilicity. In another aspect, the improved property is feel. In another aspect, the improved property is texture. In another aspect, the improved property is hand feel properties. In another aspect, the improved property is magnetic properties. In another aspect, the improved property is rheology. In another aspect, the improved property is compositional properties.

### Polymeric Xyloglucan

In the methods of the present invention, the polymeric xyloglucan can be any xyloglucan. In one aspect, the polymeric xyloglucan is obtained from natural sources. In another aspect, the polymeric xyloglucan is synthesized from component carbohydrates, UDP- or GDP-carbohydrates, or halogenated carbohydrates by any means used by those skilled in the art. In another aspect, the natural source of polymeric xyloglucan is tamarind seed or tamarind kernel powder, nasturtium, or plants of the genus *Tropaeolum,* particularly *Tropaeolum majus.* The natural source of polymeric xyloglucan may be seeds of various dicotyledonous plants such as *Hymenaea courbaril,* Leguminosae-Caesalpinioideae including the genera Cynometreae, Amherstieae, and Sclerolobieae. The natural source of polymeric xyloglucan may also be the seeds of plants of the families Primulales, Annonaceae, Limnanthaceae, Melianthaceae, Pedaliaceae, and Tropaeolaceae or subfamily Thunbergioideae. The natural source of polymeric xyloglucan may also be the seeds of plants of the families Balsaminaceae, Acanthaceae, Linaceae, Ranunculaceae, Sapindaceae, and Sapotaceae or non-endospermic members of family Leguminosae subfamily Faboideae. In another aspect, the natural source of polymeric xyloglucan is the primary cell walls of dicotyledonous plants. In another aspect, the natural source of polymeric xyloglucan may be the primary cell walls of nongraminaceous, monocotyledonous plants.

The natural source polymeric xyloglucan may be extracted by extensive boiling or hot water extraction, or by other methods known to those skilled in the art. In one aspect, the polymeric xyloglucan may be subsequently purified, for example, by precipitation in 80% ethanol. In another aspect, the polymeric xyloglucan is a crude or enriched preparation, for example, tamarind kernel powder. In another aspect, the synthetic xyloglucan may be generated by automated carbohydrate synthesis (Seeberger, Chem. Commun, 2003, 1115-1121), or by means of enzymatic polymerization, for example, using a glycosynthase (Spaduit et al., 2011, J. Am. Chem. Soc. 133: 10892-10900).

In one aspect, the average molecular weight of the polymeric xyloglucan ranges from about 2 kDa to about 500 kDa, *e.g.,* about 2 kDa to about 400 kDa, about 3 kDa to about 300 kDa, about 3 kDa to about 200 kDa, about 5 kDa to about 100 kDa, about 5 kDa to about 75 kDa, about 7.5 kDa to about 50 kDa, or about 10 kDa to about 30 kDa. In another aspect, the number of repeating units is about 2 to about 500, *e.g.,* about 2 to about 400, about 3 to about 300, about 3 to about 200, about 5 to about 100, about 7.5 to about 50, or about 10 to about 30. In another aspect, the repeating unit is any combination of G, X, L, F, S, T and J subunits, according to the nomenclature of Fry et al. (Physiologia Plantarum, 89: 1-3, 1993). In another aspect, the repeating unit is either fucosylated or non-fucosylated XXXG-type polymeric xyloglucan common to dicotyledons and nongraminaceous monocots. In another aspect, the polymeric xyloglucan is O-acetylated. In another aspect the polymeric xyloglucan is not O-acetylated. In another aspect, side chains of the polymeric xyloglucan may contain terminal fucosyl residues. In another aspect, side chains of the polymeric xyloglucan may contain terminal arabinosyl residues. In another aspect, side chains of the polymeric xyloglucan may contain terminal xylosyl residues.

For purposes of the present invention, references to the term xyloglucan herein refer to polymeric xyloglucan.

### Xyloglucan Oligomer

In the methods of the present invention, the xyloglucan oligomer can be any xyloglucan oligomer. The xyloglucan oligomer may be obtained by degradation or hydrolysis of polymeric xyloglucan from any source. The xyloglucan oligomer may be obtained by enzymatic degradation of polymeric xyloglucan, *e.g.,* by quantitative or partial digestion with a xyloglucanase or endoglucanase (endo-β-1-4-glucanase). The xyloglucan oligomer may be synthesized from component carbohydrates, UDP- or GDP-carbohydrates, or halogenated carbohydrates by any of the manners commonly used by those skilled in the art.

In one aspect, the average molecular weight of the xyloglucan oligomer ranges from 0.5 kDa to about 500 kDa, *e.g.,* about 1 kDa to about 20 kDa, about 1 kDa to about 10 kDa, or about 1 kDa to about 3 kDa. In another aspect, the number of repeating units is about 1 to about 500, *e.g.,* about 1 to about 20, about 1 to about 10, or about 1 to about 3. In the methods of the present invention, the xyloglucan oligomer is optimally as short as possible (*i.e.,* 1 repeating unit, or about 1 kDa in molecular weight) to maximize the solubility and solution molarity per gram of dissolved xyloglucan oligomer, while maintaining substrate specificity for xyloglucan endotransglycosylase activity. In another aspect, the xyloglucan oligomer comprises any combination of G (β-D glucopyranosyl-), X (α-D-xylopyranosyl-(1→6)-β-D-glucopyranosyl-), L (β-D-galactopyranosyl-(1→2)-α-D-xylopyranosyl-(1→6)-β-D-glucopyranosyl-), F (α-L-fuco-pyranosyl-(1→2)-β-D-galactopyranosyl-(1→2)-α-D-xylopyranosyl-(1→6)-β-D-glucopyranosyl-), S (α-L-arabinofurosyl-(1→2)-α-D-xylopyranosyl-(1→6)-β-D-glucopyranosyl-), T (α-L-arabino-furosyl-(1→3)-α-L-arabinofurosyl-(1→2)-α-D-xylopyranosyl-(1→6)-β-D-glucopyranosyl-), and J (α-L-galactopyranosyl-(1→2)-β-D-galactopyranosyl-(1→2)-α-D-xylopyranosyl-(1→6)-β-D-gluco-pyranosyl-) subunits according to the nomenclature of Fry et al. (Physiologia Plantarum 89: 1-3, 1993). In another aspect, the xyloglucan oligomer is the XXXG heptasaccharide common to dicotyledons and nongraminaceous monocots. In another aspect, the xyloglucan oligomer is O-acetylated. In another aspect, the xyloglucan oligomer is not O-acetylated. In another aspect, side chains of the xyloglucan oligomer may contain terminal fucosyl residues. In another aspect, side chains of the xyloglucan oligomer may contain terminal arabinosyl residues. In another aspect, side chains of the xyloglucan oligomer may contain terminal xylosyl residues.

### Functionalization of Xyloglucan Oligomer and Polymeric Xyloglucan

The xyloglucan oligomer can be functionalized by incorporating any chemical group known to those skilled in the art. The chemical group may be a compound of interest or a reactive group such as an aldehyde group, an amino group, an aromatic group, a carboxyl group, a halogen group, a hydroxyl group, a ketone group, a nitrile group, a nitro group, a sulfhydryl group, or a sulfonate group.

In one aspect, the chemical group is an aldehyde group.

In another aspect, the chemical group is an amino group. The amino group can be incorporated into polymeric xyloglucan by reductive amination. Alternatively, the amino group can be an aliphatic amine or an aromatic amine (e.g., aniline). The aliphatic amine can be a primary, secondary or tertiary amine. Primary, secondary, and tertiary amines are nitrogens bound to one, two and three carbons, respectively. In one aspect, the primary amine is C₁-C₈, *e.g.,* ethylamine. In another aspect, each carbon in the secondary amine is C₁-C₈, *e.g.,* diethylamine. In another aspect, each carbon in the tertiary amine is C₁-C₈, *e.g.,* triethyamine.

In another aspect, the chemical group is an aromatic group. The aromatic group can be an arene group, an aryl halide group, a phenolic group, a phenylamine group, a diazonium group, or a heterocyclic group.

In another aspect, the chemical group is a carboxyl group. The carboxyl group can be an acyl halide, an amide, a carboxylic acid, an ester, or a thioester.

In another aspect, the chemical group is a halogen group. The halogen group can be fluorine, chlorine, bromine, or iodine.

In another aspect, the chemical group is a hydroxyl group.

In another aspect, the chemical group is a ketone group.

In another aspect, the chemical group is a nitrile group.

In another aspect, the chemical group is a nitro group.

In another aspect, the chemical group is a sulfhydryl group.

In another aspect, the chemical group is a sulfonate group.

The chemical reactive group can itself be the chemical group that imparts an improved property to a material.

By incorporation of chemical reactive groups in such a manner, one skilled in the art can further derivatize the incorporated reactive groups with compounds (*e.g.,* macromolecules) that will impart a desired physical or chemical property to a material. For example, the incorporated chemical group may react with the compound that imparts the desired property to incorporate that group into the xyloglucan oligomer via a covalent bond. Alternatively, the chemical group may bind to the compound that imparts the desired property in either a reversible or irreversible manner, and incorporate the compound via a non-covalent association. The derivatization can be performed directly on the functionalized xyloglucan oligomer or after the functionalized xyloglucan oligomer is incorporated into polymeric xyloglucan.

Alternatively, the xyloglucan oligomer can be functionalized by incorporating directly a compound that imparts a desired physical or chemical property to a material by using a reactive group contained in the compound or a reactive group incorporated into the compound, such as any of the groups described above.

On the other hand, the polymeric xyloglucan can be directly functionalized by incorporating a reactive chemical group as described above. By incorporation of reactive chemical groups directly into polymeric xyloglucan, one of skill in the art can further derivatize the incorporated reactive groups with compounds that will impart a desired physical or chemical property to a material. By incorporation of a compound directly into the polymeric xyloglucan, a desired physical or chemical property can also be directly imparted to a material.

In one aspect, the functionalization is performed by reacting the reducing end hydroxyl of the xyloglucan oligomer or the polymeric xyloglucan. In another aspect, a non-reducing hydroxyl group, other than the non-reducing hydroxyl at position 4 of the terminal glucose, can be reacted. In another aspect, the reducing end hydroxyl and a non-reducing hydroxyl, other than the non-reducing hydroxyl at position 4 of the terminal glucose, can be reacted.

The chemical functional group can be added by enzymatic modification of the xyloglucan oligomer or polymeric xyloglucan, or by a non-enzymatic chemical reaction. In one aspect, enzymatic modification is used to add the chemical functional group. In one embodiment of enzymatic modification, the enzymatic functionalization is oxidation to a ketone or carboxylate, *e.g.,* by galactose oxidase. In another embodiment of enzymatic modification, the enzymatic functionalization is oxidation to a ketone or carboxylate by AA9 Family oxidases (formerly glycohydrolase Family 61 enzymes).

In another aspect, the chemical functional group is added by a non-enzymatic chemical reaction. In one embodiment of the non-enzymatic chemical reaction, the reaction is incorporation of a reactive amine group by reductive amination of the reducing end of the carbohydrate as described by Roy et al., 1984, Can. J. Chem. 62: 270-275, or Dalpathado et al., 2005, Anal. Bioanal. Chem. 381: 1130-1137. In another embodiment of non-enzymatic chemical reaction, the reaction is incorporation of a reactive ketone group by oxidation of the reducing end hydroxyl to a ketone, *e.g.,* by copper (II). In another embodiment of non-enzymatic chemical reaction, the reaction is oxidation of non-reducing end hydroxyl groups (*e.g.,* of the non-glycosidic bonded position 6 hydroxyls of glucose or galactose) by (2,2,6,6-tetramethyl-piperidin-1-yl)oxyl (TEMPO), or the oxoammonium salt thereof, to generate an aldehyde or carboxylic acid as described in Bragd et al., 2002, Carbohydrate Polymers 49: 397-406, or Breton et al., 2007, Eur. J. Org. Chem. 10: 1567-1570.

Xyloglucan oligomers or polymeric xyloglucan can be functionalized by a chemical reaction with a compound containing more than one (*i.e.* bifunctional or multifunctional) chemical functional group comprising at least one chemical functional group that is directly reactive with xyloglucan oligomer or polymeric xyloglucan. In one aspect, the bifunctional chemical group is a hydrocarbon containing a primary amine and a second chemical functional group. The second functional group can be any of the other groups described above. In some aspects, the two functional groups are separated by hydrocarbon chains (linkers) of various lengths as is well known in the art.

Xyloglucan oligomers or polymeric xyloglucan can be functionalized with a compound of interest by step-wise or concerted reaction wherein the xyloglucan oligomer or polymeric xyloglucan is functionalized as described above, and the compound is reactive to the functionalization introduced therein. In one aspect of coupling via a functionalized xyloglucan oligomer, an amino group is first incorporated into the xyloglucan oligomer by reductive amination and a reactive carbonyl is secondarily coupled to the introduced amino group. In another aspect of coupling via an amino-modified xyloglucan oligomer, the second coupling step incorporates a chemical group, compound or macromolecule via coupling an N-hydroxysuccinimidyl (NHS) ester or imidoester to the introduced amino group. In a preferred embodiment, the NHS ester secondarily coupled to the introduced amino group is a component of a mono or bi-functional crosslink reagent. In another aspect of coupling to a functionalized xyloglucan or xyloglucan oligomer, the first reaction step comprises functionalization with a sulfhydryl group, either via reductive amination with an alkylthioamine (NH₂-(CH₂)ₙ-SH) at elevated temperatures in the presence of a reducing agent (Magid et al., 1996, J. Org. Chem. 61: 3849-3862), or via radical coupling (Wang et al., 2009, Arkivoc xiv: 171-180), followed by reaction of a maleimide group to the sulfhydryl. In some aspects, the reactive group in the compound that imparts the desired property is separated from the rest of the compound by a hydrocarbon chain of an appropriate length, as is well described in the art.

Non-limiting examples of compounds of interest that can be used to functionalize polymeric xyloglucan or xyloglucan oligomers, either by direct reaction or via reaction with a xyloglucan-reactive compound, include peptides, polypeptides, proteins, hydrophobic groups, hydrophilic groups, flame retardants, dyes, color modifiers, specific affinity tags, non-specific affinity tags, metals, metal oxides, metal sulfides, minerals, fungicides, herbicides, microbicides or microbiostatics, and non-covalent linker molecules.

In one aspect, the compound is a peptide. The peptide can be an antimicrobial peptide, a "self-peptide" designed to reduce allergenicity and immunogenicity, a cyclic peptide, glutathione, or a signaling peptide (such as a tachykinin peptide, vasoactive intestinal peptide, pancreatic polypeptide related peptide, calcitonin peptide, lipopeptide, cyclic lipopeptide, or other peptide).

In another aspect, the compound is a polypeptide. The polypeptide can be a non-catalytically active protein (*i.e.,* structural or binding protein), or a catalytically active protein (*i.e.,* enzyme). The polypeptide can be an enzyme, an antibody, or an abzyme.

In another aspect, the compound is a compound comprising a hydrophobic group. The hydrophobic group can be polyurethane, polytetrafluoroethylene, or polyvinylidene fluoride.

In another aspect, the compound is a compound comprising a hydrophilic group. The hydrophilic group can be methacylate, methacrylamide, or polyacrylate.

In another aspect, the compound is a flame retardant. The flame-retardant can be aluminum hydroxide or magnesium hydroxide. The flame-retardant can also be a compound comprising an organohalogen group or an organophosphorous group.

In another aspect, the compound is a dye or pigment.

In another aspect, the compound is a specific affinity tag. The specific affinity tag can be biotin, avidin, a chelating group, a crown ether, a heme group, a non-reactive substrate analog, an antibody, target antigen, or a lectin.

In another aspect, the compound is a non-specific affinity tag. The non-specific affinity tag can be a polycation group, a polyanion group, a magnetic particle (*e.g.,* magnetite), a hydrophobic group, an aliphatic group, a metal, a metal oxide, a metal sulfide, or a molecular sieve.

In another aspect, the compound is a fungicide. The fungicide can be a compound comprising a dicarboximide group (such as vinclozolin), a phenylpyrrole group (such as fludioxonil), a chlorophenyl group (such as quintozene), a chloronitrobenzene (such as dicloran), a triadiazole group (such as etridiazole), a dithiocarbamate group (such as mancozeb or dimethyldithiocarbamate), or an inorganic molecule (such as copper or sulfur). In another aspect, the fungicide is a bacterium or bacterial spore such as *Bacillus* or a *Bacillus* spore.

In another aspect, the compound is a herbicide. The herbicide can be glyphosate, a synthetic plant hormone (such as a compound comprising a 2,4-dichloropenoxyacetic acid group, a 2,4,5-trichlorophenoxyacetic acid group, a 2-methyl-4-chlorophenoxyacetic acid group, a 2-(2-methyl-4-chlorophenoxy)propionic acid group, a 2-(2,4-dichlorophenoxy)propionic acid group, or a (2,4-dichlorophenoxy)butyric acid group), or a compound comprising a triazine group (such as atrazine (2-chloro-4-(ethylamino)-6-isopropylamino)-s-triazine).

In another aspect, the compound is a bactericidal or bacteriostatic compound. The bactericidal or bacteriostatic compound can be a copper or copper alloy (such as brass, bronze, cupronickel, or copper-nickel-zinc alloy), a sulfonamide group (such as sulfamethoxazole, sulfisomidine, sulfacetamide or sulfadiazine), a silver or organo-silver group, TiO₂, ZnO₂, an antimicrobial peptide, or chitosan.

In another aspect, the compound is a non-covalent linker molecule.

In another aspect, the compound is a color modifier. The color modifier can be a dye, fluorescent brightener, color modifier, or mordant (*e.g.,* alum, chrome alum).

In another aspect, the compound is a metal.

In another aspect, the compound is a semi-conductor. The semi-conductor can be an organic semi-conductor, a binary or ternary compound, or a semi-conducting element.

In another aspect, the compound is a UV-resistant compound. The UV resistant compound can be zinc or ZnO₂, kaolin, aluminum, aluminum oxides, or other UV-resistant compounds.

In another aspect, the compound is an anti-oxidant compound. The anti-oxidant compound can be ascorbate, manganese, iodide, retinol, a terpenoid, tocopherol, a flavonoid or other anti-oxidant phenolic or polyphenolic or other anti-oxidant compounds.

### Preparation of Functionalized and Composite Materials

In the methods of the present invention, a functionalized material can be prepared from any material. The material can be functionalized by treating the material with (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; (h) a composition comprising a xyloglucan endotransglycosylase and a xyloglucan oligomer, or (i) a composition of (a), (b), (c), (d), (e), (f), (g), or (h) without a xyloglucan endotransglycosylase, under conditions leading to a modified material, wherein the modified material possesses an improved property compared to the unmodified material.

A linked or composite material can be prepared by treating two or more materials with a composition comprising a xyloglucan endotransglycosylase, a polymeric xyloglucan, and a functionalized xyloglucan oligomer comprising a chemical group, under conditions leading to association between the two or more materials. A linked or composite material can also be prepared by treating two or more materials with a composition comprising a xyloglucan endotransglycosylase, a polymeric xyloglucan functionalized with a chemical group, and a functionalized xyloglucan oligomer comprising a chemical group, under conditions leading to association between the two or more materials. A linked or composite material can also be prepared by treating two or more materials with a composition comprising a xyloglucan endotransglycosylase, a polymeric xyloglucan functionalized with a chemical group, and a xyloglucan oligomer, under conditions leading to association between the two or more materials. A linked or composite material can also be prepared by treating two or more materials with a composition comprising a xyloglucan endotransglycosylase, a polymeric xyloglucan, and a xyloglucan oligomer, under conditions leading to association between the two or more materials. A linked or composite material can also be prepared by treating two or more materials with a composition comprising a xyloglucan endotransglycosylase and a polymeric xyloglucan functionalized with a chemical group, under conditions leading to association between the two or more materials. A linked or composite material can also be prepared by treating two or more materials with a composition comprising a xyloglucan endotransglycosylase and a polymeric xyloglucan, under conditions leading to association between the two or more materials. A linked or composite material can also be prepared by treating two or more materials with a composition comprising a xyloglucan endotransglycosylase and a functionalized xyloglucan oligomer comprising a chemical group, under conditions leading to association between the two or more materials. A linked or composite material can also be prepared by treating two or more materials with a composition comprising a xyloglucan endotransglycosylase and a xyloglucan oligomer, under conditions leading to association between the two or more materials.

The methods are exemplified by, but are not limited to, enhanced association of silica (SiO₂) or titanium dioxide (TiO₂) with plastics, permitting direct binding of the constituent materials. In the methods of the present invention, a slurry of titanium dioxide can be incubated in a pH controlled solution, *e.g.,* buffered solution (*e.g.,* sodium citrate) from pH 3 to pH 9, *e.g.,* from pH 4 to pH 8 or from pH 5 to pH 7, at concentrations from about 1 g/L to about 10 kg/L, *e.g.,* about 10 g/L to about 1 kg/L or about 40 g/L to about 100 g/L containing xyloglucan endotransglycosylase and polymeric xyloglucan with or without functionalized xyloglucan oligomer. The xyloglucan endotransglycosylase can be present at about 0.1 nM to about 1 mM, *e.g.,* about 10 nM to about 100 µM or about 0.5 µM to about 5 µM. In one aspect, the xyloglucan endotransglycosylase is present at a concentration of 320 pg to about 32 mg of enzyme per g of the constituent mineral material, *e.g.,* about 160 µg to about 4 mg of enzyme per g of the constituent mineral material. When present, the functionalized xyloglucan oligomer can be present with polymeric xyloglucan at about 50:1 to about 0.5:1 molar ratio of functionalized xyloglucan oligomer to polymeric xyloglucan, *e.g.,* about 10:1 to about 1:1 or about 5:1 to about 1:1 molar ratio of functionalized xyloglucan oligomer to polymeric xyloglucan. The polymeric xyloglucan can be present at about 1 mg per g of the constituent mineral material to about 1 g per g of the constituent mineral material, *e.g.,* about 10 mg to about 100 mg per g of the constituent mineral material or about 20 mg to about 50 mg per g of the constituent mineral material. Second or additional constituent materials can be matrix constituents, skin constituents, core constituents, laminate constituents, resin constituents or reinforcement constituent materials. In the present non-limiting example, the second constituent material is polystyrene. In one aspect, the polystyrene is incubated in buffered solution with the polymeric xyloglucan-modified TiO₂ or SiO₂ in the presence of xyloglucan endotransglycosylase. In another aspect, the polystyrene is added to the TiO₂ or SiO₂ incubation with polymeric xyloglucan and xyloglucan endotransglycosylase described above. In another aspect, the polystyrene is heated above the glass transition temperature in the presence of the polymeric xyloglucan-modified TiO₂ or SiO₂, formed and allowed to cool. In another aspect the polystyrene is formed prior to addition of TiO₂ or SiO₂. The mass ratio of TiO₂ to polystyrene in the composite can be from about 0.0001 to 1, *e.g.* about 0.01 to 0.5 or about 0.1 to about 0.3. The incubation can last for a sufficient period of time to effect the desired extent of association, *e.g.,* about instantaneously to about 72 hours, about 15 minutes to about 48 hours, about 30 minutes to about 24 hours, or about 1 to about 3 hours. In one aspect, the constituent mineral material is separated from xyloglucan endotransglycosylase and unbound polymeric xyloglucan or functionalized xyloglucan oligomer by washing in, for example, water. In another aspect of the present invention, the constituent mineral material is then dried.

In one aspect of the present invention, the polymeric xyloglucan is functionalized prior to functionalization of the constituent materials. The polymeric xyloglucan can be incubated in pH controlled solution with xyloglucan endotransglycosylase and functionalized xyloglucan oligomers, yielding polymeric xyloglucan functionalized with a chemical group. Polymeric xyloglucan functionalized with a chemical group can then be separated from functionalized xyloglucan oligomers by any method known in the art, but as exemplified by ethanol precipitation. For example, the reaction mixture can be incubated in 80% (v/v) ethanol for about 1 minute to about 24 hours, *e.g.,* 30 minutes to 20 hours or 12 to 15 hours, centrifuged for an appropriate length of time at an appropriate velocity to pellet the precipitated, polymeric xyloglucan functionalized with a chemical group (*e.g.,* 30 minutes at approximately 2000 x *g*), and the supernatants decanted. The polymeric xyloglucan functionalized with a chemical group is then optionally dried. In this aspect of the present invention, the polymeric xyloglucan functionalized with a chemical group is then incubated with xyloglucan endotransglycosylase and the filler material.

### Sources of Xyloglucan Endotransglycosylases

Any xyloglucan endotransglycosylase that possesses suitable enzyme activity at a pH and temperature appropriate for the methods of the present invention may be used. It is preferable that the xyloglucan endotransglycosylase is active over a broad pH and temperature range. In an embodiment, the xyloglucan endotransglycosylase has a pH optimum in the range of about 3 to about 10. In another embodiment, the xyloglucan endotransglycosylase has a pH optimum in the range of about 4.5 to about 8.5. In another embodiment, the xyloglucan endotransglycosylase has a cold denaturation temperature less than or equal to about 5°C or a melting temperature of about 100°C or higher. In another embodiment, the xyloglucan endotransglycosylase has a cold denaturation temperature of less than or equal to 20°C or a melting temperature greater than or equal to about 75°C.

The source of the xyloglucan endotransglycosylase used is not critical in the present invention. Accordingly, the xyloglucan endotransglycosylase may be obtained from any source such as a plant, microorganism, or animal.

In one embodiment, the xyloglucan endotransglycosylase is obtained from a plant source. Xyloglucan endotransglycosylase can be obtained from cotyledons of the family Fabaceae (synonyms: Leguminosae and Papilionaceae), preferably genus *Phaseolus,* in particular, *Phaseolus aureus.* Preferred monocotyledons are non-graminaceous monocotyledons and liliaceous monocotyledons. Xyloglucan endotransglycosylase can also be extracted from moss and liverwort, as described in Fry et al., 1992, Biochem. J. 282: 821-828. For example, the xyloglucan endotransglycosylase may be obtained from cotyledons, *i.e.,* a dicotyledon or a monocotyledon, in particular a dicotyledon selected from the group consisting of azuki beans, canola, cauliflowers, cotton, poplar or hybrid aspen, potatoes, rapes, soy beans, sunflowers, thalecress, tobacco, and tomatoes, or a monocotyledon selected from the group consisting of wheat, rice, corn, and sugar cane. See, for example, WO 2003/033813 and WO 97/23683.

In another embodiment, the xyloglucan endotransglycosylase is obtained from *Arabidopsis thaliana* (GENESEQP:AOE11231, GENESEQP:AOE93420, GENESEQP: BAL03414, GENESEQP:BAL03622, or GENESEQP:AWK95154); *Carica papaya* (GENESEQP:AZR75725); *Cucumis sativus* (GENESEQP:AZV66490); *Daucus carota* (GENESEQP:AZV66139); *Festuca pratensis* (GENESEQP:AZR80321); *Glycine max* (GENESEQP:AWK95154 or GENESEQP:AYF92062); *Hordeum vulgare* (GENESEQP:AZR85056, GENESEQP:AQY12558, GENESEQP:AQY12559, or GENESEQP:AWK95180); Lycopersicon esculentum (GENESEQP:ATZ45232); *Medicago truncatula* (GENESEQP:ATZ48025); *Oryza sativa* (GENESEQP:ATZ42485, GENESEQP:ATZ57524, or GENESEQP:AZR76430); *Populus tremula* (GENESEQP:AWK95036); *Sagittaria pygmaea* (GENESEQP:AZV66468); *Sorghum bicolor* (GENESEQP:BAO79623 or GENESEQP:BAO79007); *Vigna angularis* (GENESEQP:ATZ61320); or *Zea mays* (GENESEQP:AWK94916).

In another embodiment, the xyloglucan endotransglycosylase is a xyloglucan endotransglucosylase/hydrolase (XTH) with both hydrolytic and transglycosylating activities. In a preferred embodiment, the ratio of transglycosylation to hydrolytic rates is at least 10⁻² to 10⁷, *e.g.,* 10⁻¹ to 10⁶ or 10 to 1000.

### Production of Xyloglucan Endotransglycosylases

Xyloglucan endotransglycosylase may be extracted from plants. Suitable methods for extracting xyloglucan endotransglycosylase from plants are described Fry et al., 1992, Biochem. J. 282: 821-828; Sulova et al., 1998, Biochem. J. 330: 1475-1480; Sulova et al., 1995, Anal. Biochem. 229: 80-85; WO 95/13384; WO 97/23683; or EP 562 836.

Xyloglucan endotransglycosylase may also be produced by cultivation of a transformed host organism containing the appropriate genetic information from a plant, microorganism, or animal. Transformants can be prepared and cultivated by methods known in the art.

Techniques used to isolate or clone a gene are known in the art and include isolation from genomic DNA or cDNA, or a combination thereof. The cloning of the gene from genomic DNA can be effected, *e.g.,* by using the polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used.

A nucleic acid construct can be constructed to comprise a gene encoding a xyloglucan endotransglycosylase operably linked to one or more control sequences that direct the expression of the coding sequence in a suitable host cell under conditions compatible with the control sequences. The gene may be manipulated in a variety of ways to provide for expression of the xyloglucan endotransglycosylase. Manipulation of the gene prior to its insertion into a vector may be desirable or necessary depending on the expression vector. Techniques for modifying polynucleotides utilizing recombinant DNA methods are well known in the art.

The control sequence may be a promoter, a polynucleotide that is recognized by a host cell for expression of a polynucleotide encoding a xyloglucan endotransglycosylase. The promoter contains transcriptional control sequences that mediate the expression of the xyloglucan endotransglycosylase. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the xyloglucan endotransglycosylase. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the xyloglucan endotransglycosylase. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a xyloglucan endotransglycosylase and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*)*,* and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a xyloglucan endotransglycosylase. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a xyloglucan endotransglycosylase and the signal peptide sequence is positioned next to the N-terminus of the propeptide sequence.

The various nucleotide and control sequences may be joined together to produce a recombinant expression vector that may include one or more convenient restriction sites to allow for insertion or substitution of the polynucleotide encoding the xyloglucan endotransglycosylase at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, adeA (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus* bar gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the xyloglucan endotransglycosylase or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide may be inserted into a host cell to increase production of a xyloglucan endotransglycosylase. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors are well known to one skilled in the art (see, *e.g.,* Sambrook *et al.,* 1989, *supra*).

The host cell may be any cell useful in the recombinant production of a xyloglucan endotransglycosylase, *e.g.,* a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong etal., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al.,* 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp. 182-187, Academic Press, Inc., New York; Ito et al., 1983, J. Bacteriol. 153: 163; and Hinnen et al., 1978, Proc. Natl. Acad. Sci. USA 75: 1920.

The host cells are cultivated in a nutrient medium suitable for production of the xyloglucan endotransglycosylase using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the xyloglucan endotransglycosylase to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the xyloglucan endotransglycosylase is secreted into the nutrient medium, the polypeptide can be recovered directly from the medium. If the xyloglucan endotransglycosylase is not secreted, it can be recovered from cell lysates.

The xyloglucan endotransglycosylase may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The xyloglucan endotransglycosylase may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a whole fermentation broth comprising the polypeptide is recovered. In a preferred aspect, xyloglucan endotransglycosylase yield may be improved by subsequently washing cellular debris in buffer or in buffered detergent solution to extract biomass-associated polypeptide.

The xyloglucan endotransglycosylase may be purified by a variety of procedures known in the art including, but not limited to, chromatography (*e.g.,* ion exchange, affinity, hydrophobic interaction, mixed mode, reverse phase, chromatofocusing, and size exclusion), electrophoretic procedures (*e.g.,* preparative isoelectric focusing), differential solubility (*e.g.,* ammonium sulfate precipitation), PAGE, membrane-filtration or extraction (see, *e.g.,* Protein Purification, Janson and Ryden, editors, VCH Publishers, New York, 1989) to obtain substantially pure polypeptide. In a preferred aspect, xyloglucan endotransglycosylase may be purified by formation of a covalent acyl-enzyme intermediate with xyloglucan, followed by precipitation with microcrystalline cellulose or adsorption to cellulose membranes. Release of the polypeptide is then effected by addition of xyloglucan oligomers to resolve the covalent intermediate (Sulova and Farkas, 1999, Protein Expression and Purification 16(2): 231-235, and Steele and Fry, 1999, Biochemical Journal 340: 207-211).

The present invention is further described by the following examples that should not be construed as limiting the scope of the invention.

### Examples

### Media and Solutions

COVE agar plates were composed of 342.3 g of sucrose, 252.54 g of CsCI, 59.1 g of acetamide, 520 mg of KCl, 520 mg of MgSO₄·7H₂O, 1.52 g of KH₂PO₄, 0.04 mg of Na₂B₄O₇·10H₂O, 0.4 mg of CuSO₄·5H₂O, 1.2 mg of FeSO₄·7H₂O, 0.7 mg of MnSO₄·2H₂O, 0.8 mg of Na₂MoO₄·2H₂O, 10 mg of ZnSO₄·7H₂O, 25 g of Noble agar, and deionized water to 1 liter.

LB medium was composed of 10 g of tryptone, 5 g of yeast extract, 5 g of NaCl, and deionized water to 1 liter.

LB plates were composed of 10 g of tryptone, 5 g of yeast extract, 5 g of NaCl, 15 g of bacteriological agar, and deionized water to 1 liter.

Minimal medium agar plates were composed of 342.3 g of sucrose, 10 g of glucose, 4 g of MgSO₄·7H₂O, 6 g of NaNO₃, 0.52 g of KCl, 1.52 g of KH₂PO₄, 0.04 mg of Na₂B₄O₇·10H₂O, 0.4 mg of CuSO₄·5H₂O, 1.2 mg of FeSO₄·7H₂O, 0.7 mg of MnSO₄·2H₂O, 0.8 mg of Na₂MoO₄·2H₂O, 10 mg of ZnSO₄·7H₂O, 500 mg of citric acid, 4 mg of d-biotin, 20 g of Noble agar, and deionized water to 1 liter.

Synthetic defined medium lacking uridine was composed of 18 mg of adenine hemisulfate, 76 mg of alanine, 76 mg of arginine hydrochloride, 76 mg of asparagine monohydrate, 76 mg of aspartic acid, 76 mg of cysteine hydrochloride monohydrate, 76 mg of glutamic acid monosodium salt, 76 mg of glutamine, 76 mg of glycine, 76 mg of histidine, myo-76 mg of inositol, 76 mg of isoleucine, 380 mg of leucine, 76 mg of lysine monohydrochloride, 76 mg of methionine, 8 mg of p-aminobenzoic acid potassium salt, 76 mg of phenylalanine, 76 mg of proline, 76 mg of serine, 76 mg of threonine, 76 mg of tryptophan, 76 mg of tyrosine disodium salt, 76 mg of valine, and deionized water to 1 liter.

TAE buffer was composed of 4.84 g of Tris base, 1.14 ml of glacial acetic acid, 2 ml of 0.5 M EDTA pH 8.0, and deionized water to 1 liter.

TBE buffer was composed of 10.8 g of Tris base, 5.5 g of boric acid, 4 ml of 0.5 M EDTA pH 8.0, and deionized water to 1 liter.

2XYT plus ampicillin plates were composed of 16 g of tryptone, 10 g of yeast extract, 5 g of sodium chloride, 15 g of Bacto agar, and deionized water to 1 liter. One ml of a 100 mg/ml solution of ampicillin was added after the autoclaved medium was tempered to 55°C.

YP + 2% glucose medium was composed of 10 g of yeast extract, 20 g of peptone, 20 g of glucose, and deionized water to 1 liter.

YP + 2% maltodextrin medium was composed of 10 g of yeast extract, 20 g of peptone, 20 g of maltodextrin, and deionized water to 1 liter.

### Example 1: Preparation of Vigna angularis xyloglucan endotransglycosylase 16

*Vigna angularis* xyloglucan endotransglycosylase 16 (VaXET16; SEQ ID NO: 1 [native DNA sequence], SEQ ID NO: 2 [synthetic DNA sequence], and SEQ ID NO: 3 [deduced amino acid sequence]; also referred to as XTH1) was recombinantly produced in *Aspergillus oryzae* MT3568 according to the protocol described below. *Aspergillus oryzae* MT3568 is an *amdS* (acetamidase) disrupted gene derivative of *Aspergillus oryzae* JaL355 (WO 2002/40694), in which *pyrG* auxotrophy was restored by disrupting the *A. oryzae amdS* gene with the *pyrG* gene.

The vector pDLHD0012 was constructed to express the VaXET16 gene in multi-copy in *Aspergillus oryzae.* Plasmid pDLHD0012 was generated by combining two DNA fragments using megaprimer cloning: Fragment 1 containing the VaXET16 ORF and flanking sequences with homology to vector pBM120 (US20090253171), and Fragment 2 consisting of an inverse PCR amplicon of vector pBM120.

Fragment 1 was amplified using primer 613788 (sense) and primer 613983 (antisense) shown below. These primers were designed to contain flanking regions of sequence homology to vector pBM120 (lower case) for ligation-free cloning between the PCR fragments.
Primer 613788 (sense):
   ttcctcaatcctctatatacacaactggccATGGGCTCGTCCCTCTGGAC (SEQ ID NO: 7)
Primer 613983 (antisense):
   tgtcagtcacctctagttaattaGATGTCCCTATCGCGTGTACACTCG (SEQ ID NO: 8)

Fragment 1 was amplified by PCR in a reaction composed of 10 ng of a GENEART® vector pMA containing the VaXET16 synthetic gene (SEQ ID NO: 3 [synthetic DNA sequence]) cloned between the *Sac* I and *Kpn* I sites, 0.5 µl of PHUSION® DNA Polymerase (New England Biolabs, Inc., Ipswich, MA, USA), 20 pmol of primer 613788, 20 pmol of primer 613983, 1 µl of 10 mM dNTPs, 10 µl of 5X PHUSION® HF buffer (New England Biolabs, Inc., Ipswich, MA, USA), and 35.5 µl of water. The reaction was incubated in an EPPENDORF® MASTERCYCLER® (Eppendorf AG, Hamburg, Germany) programmed for 1 cycle at 98°C for 30 seconds; and 30 cycles each at 98°C for 10 seconds, 60°C for 10 seconds, and 72°C for 30 seconds. The resulting 0.9 kb PCR product (Fragment 1) was treated with 1 µl of *Dpn* I (Promega, Fitchburg, WI, USA) to remove plasmid template DNA. The *Dpn* I was added directly to the PCR tube, mixed well, and incubated at 37°C for 60 minutes, and then was column-purified using a MINELUTE® PCR Purification Kit (QIAGEN Inc., Valencia, CA, USA) according to the manufacturer's instructions.

Fragment 2 was amplified using primers 613786 (sense) and 613787 (antisense) shown below.
613786 (sense):
   taattaactagaggtgactgacacctggc (SEQ ID NO: 9)
613787 (antisense):
   catggccagttgtgtatatagaggattgagg (SEQ ID NO: 10)

Fragment 2 was amplified by PCR in a reaction composed of 10 ng of plasmid pBM120, 0.5 µl of PHUSION® DNA Polymerase, 20 pmol of primer 613786, 20 pmol of primer 613787, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; and 30 cycles each at 98°C for 10 seconds, 60°C for 10 seconds, and 72°C for 4 minutes. The resulting 6.9 kb PCR product (Fragment 2) was treated with 1 µl of *Dpn* I to remove plasmid template DNA. The *Dpn* I was added directly to the PCR tube, mixed well, and incubated at 37°C for 60 minutes, and then column-purified using a MINELUTE® PCR Purification Kit according to the manufacturer's instructions.

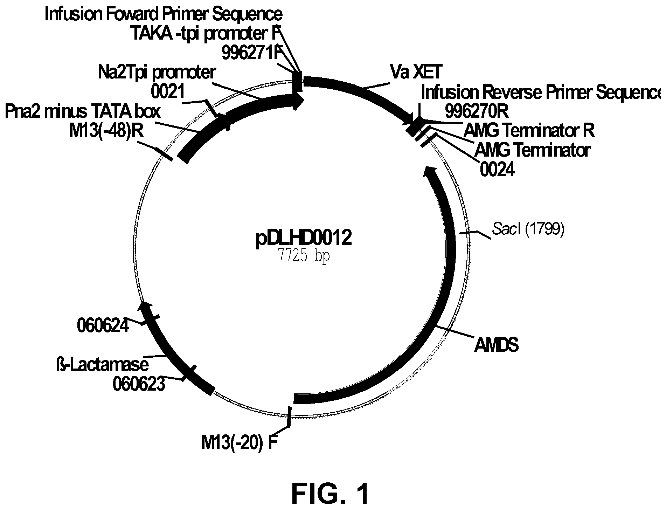

The following procedure was used to combine the two PCR fragments using megaprimer cloning. Fragments 1 and 2 were combined by PCR in a reaction composed of 5 µl of each purified PCR product, 0.5 µl of PHUSION® DNA Polymerase, 1 µl of 10 mM dNTPs, 10 µ! of 5X PHUSION® HF buffer, and 28.5 µl of water. The reaction was incubated in an EPPENDORF® MASTERCYCLER® programmed for 1 cycle at 98°C for 30 seconds; and 40 cycles each at 98°C for 10 seconds, 60°C for 10 seconds, and 72°C for 4 minutes. Two µl of the resulting PCR product DNA was then transformed into *E. coli* ONE SHOT® TOP10 electrocompetent cells (Life Technologies, Grand Island, NY, USA) according the manufacturer's instructions. Fifty µl of transformed cells were spread onto LB plates supplemented with 100 µg of ampicillin per ml and incubated at 37°C overnight. Individual transformants were picked into 3 ml of LB medium supplemented with 100 µg of ampicillin per ml and grown overnight at 37°C with shaking at 250 rpm. The plasmid DNA was purified from the colonies using a QIAPREP® Spin Miniprep Kit (QIAGEN Inc., Valencia, CA, USA). DNA sequencing using a 3130XL Genetic Analyzer (Applied Biosystems, Foster City, CA, USA) was used to confirm the presence of each of both fragments in the final plasmid pDLHD0012 (Figure 1).

*Aspergillus oryzae* strain MT3568 was transformed with plasmid pDLHD0012 comprising the VaXET16 gene according to the following protocol. Approximately 2-5 x 10⁷ spores of *A. oryzae* strain MT3568 were inoculated into 100 ml of YP + 2% glucose medium in a 500 ml shake flask and incubated at 28°C and 110 rpm overnight. Ten ml of the overnight culture were filtered in a 125 ml sterile vacuum filter, and the mycelia were washed twice with 50 ml of 0.7 M KCI-20 mM CaCl₂. The remaining liquid was removed by vacuum filtration, leaving the mat on the filter. Mycelia were resuspended in 10 ml of 0.7 M KCI-20 mM CaCl₂ and transferred to a sterile 125 ml shake flask containing 20 mg of GLUCANEX® 200 G (Novozymes Switzerland AG, Neumatt, Switzerland) per ml and 0.2 mg of chitinase (Sigma-Aldrich, St. Louis, MO, USA) per ml in 10 ml of 0.7 M KCI-20 mM CaCl₂. The mixture was incubated at 37°C and 100 rpm for 30-90 minutes until protoplasts were generated from the mycelia. The protoplast mixture was filtered through a sterile funnel lined with MIRACLOTH® (Calbiochem, San Diego, CA, USA) into a sterile 50 ml plastic centrifuge tube to remove mycelial debris. The debris in the MIRACLOTH® was washed thoroughly with 0.7 M KCI-20 mM CaCl_{2,} and centrifuged at 2500 rpm (537 x *g*) for 10 minutes at 20-23°C. The supernatant was removed and the protoplast pellet was resuspended in 20 ml of 1 M sorbitol-10 mM Tris-HCI (pH 6.5)-10 mM CaCl₂. This step was repeated twice, and the final protoplast pellet was resuspended in 1 M sorbitol-10 mM Tris-HCI (pH 6.5)-10 mM CaCl₂ to obtain a final protoplast concentration of 2 x 10⁷/ml.

Two micrograms of pDLHD0012 were added to the bottom of a sterile 2 ml plastic centrifuge tube. Then 100 µl of protoplasts were added to the tube followed by 300 µl of 60% PEG-4000 in 10 mM Tris-HCI (pH 6.5)-10 mM CaCl₂. The tube was mixed gently by hand and incubated at 37°C for 30 minutes. Two ml of 1 M sorbitol-10 mM Tris-HCI (pH 6.5)-10 mM CaCl₂ were added to each transformation and the mixture was transferred onto 150 mm COVE agar plates. Transformation plates were incubated at 34°C until colonies appeared.

Twenty-one transformant colonies were picked to fresh COVE agar plates and cultivated at 34°C for four days until the transformants sporulated. Fresh spores were transferred to 48-well deep-well plates containing 2 ml of YP + 2% maltodextrin, covered with a breathable seal, and grown for 4 days at 34°C with no shaking. After 4 days growth samples of the culture media were assayed for xyloglucan endotransglycosylase activity using an iodine stain assay and for xyloglucan endotransglycosylase expression by SDS-PAGE.

The iodine stain assay for xyloglucan endotransglycosylase activity was performed according to the following protocol. In a 96-well plate, 5 µl of culture broth were added to a mixture of 5 µl of xyloglucan (Megazyme, Bray, United Kingdom) (5 mg/ml in water), 20 µl of xyloglucan oligomers (Megazyme, Bray, United Kingdom) (5 mg/ml in water), and 10 µl of 400 mM sodium citrate pH 5.5. The reaction mix was incubated at 37°C for thirty minutes, quenched with 200 µl of a solution containing 14% (w/v) Na₂SO₄, 0.2% Kl, 100 mM HCI, and 1% iodine (I₂), incubated in the dark for 30 minutes, and then the absorbance was measured in a plate reader at 620 nm. The assay demonstrated the presence of xyloglucan endotransglycosylase activity from several transformants.

SDS-PAGE was performed using a 8-16% CRITERION® Stain Free SDS-PAGE gel (Bio-Rad Laboratories, Inc., Hercules, CA, USA), and imaging the gel with a Stain Free Imager (Bio-Rad Laboratories, Inc., Hercules, CA, USA) using the following settings: 5-minute activation, automatic imaging exposure (intense bands), highlight saturated pixels = ON, color = Coomassie, and band detection, molecular weight analysis and reporting disabled. SDS-PAGE analysis indicated that several transformants expressed a protein of approximately 32 kDa corresponding to VaXET16.

### Example 2: Construction of plasmid pMMar27 as a yeast expression plasmid vector

Plasmid pMMar27 was constructed for expression of the *T. terrestris* Cel6A cellobiohydrolase II in yeast. The plasmid was generated from a lineage of yeast expression vectors: plasmid pMMar27 was constructed from plasmid pBM175b; plasmid pBM175b was constructed from plasmid pBM143b (WO 2008/008950) and plasmid pJLin201; and plasmid pJLin201 was constructed from pBM143b.

Plasmid pJLin201 is identical to pBM143b except an *Xba* I site immediately downstream of a *Thermomyces lanuginosus* lipase variant gene in pBM143b was mutated to a unique *Nhe* I site. A QUIKCHANGE® II XL Site-Directed Mutagenesis Kit (Stratagene, La Jolla, CA, USA) was used to change the *Xba* I sequence (TCTAGA) to a *Nhe* I sequence (gCTAGc) in pBM143b. Primers employed to mutate the site are shown below.
Primer 999551 (sense):
   5'-ACATGTCTTTGATAAgCTAGcGGGCCGCATCATGTA-3' (SEQ ID NO: 11)
Primer 999552 (antisense):
   5'-TACATGATGCGGCCCgCTAGcTTATCAAAGACATGT-3' (SEQ ID NO: 12)
Lower case represents mutated nucleotides.

The amplification reaction was composed of 125 ng of each primer above, 20 ng of pBM143b, 1X QUIKCHANGE® Reaction Buffer (Stratagene, La Jolla, CA, USA), 3 µl of QUIKSOLUTION® (Stratagene, La Jolla, CA, USA), 1 µl ofdNTP mix, and 1 µl of a 2.5 units/ml *Pfu* Ultra HF DNA polymerase in a final volume of 50 µl. The reaction was performed using an EPPENDORF® MASTERCYCLER® thermocycler programmed for 1 cycle at 95°C for 1 minute; 18 cycles each at 95°C for 50 seconds, 60°C for 50 seconds, and 68°C for 6 minutes and 6 seconds; and 1 cycle at 68°C for 7 minutes. After the PCR, the tube was placed on ice for 2 minutes. One microliter of *Dpn* I was directly added to the amplification reaction and incubated at 37°C for 1 hour. A 2 µl volume of the *Dpn* I digested reaction was used to transform *E. coli* XL10-GOLD® Ultracompetent Cells (Stratagene, La Jolla, CA, USA) according to the manufacturer's instructions. *E. coli* transformants were selected on 2XYT plus ampicillin plates. Plasmid DNA was isolated from several of the transformants using a BIOROBOT® 9600. One plasmid with the desired *Nhe* I change was confirmed by restriction digestion and sequencing analysis and designated plasmid pJLin201. To eliminate possible PCR errors introduced by site-directed-mutagenesis, plasmid pBM175b was constructed by cloning the *Nhe* I site containing fragment back into plasmid pBM143b. Briefly, plasmid pJLin201 was digested with *Nde* I and *Mlu* I and the resulting fragment was cloned into pBM143b previously digested with the same enzymes using a Rapid Ligation Kit (Roche Diagnostics Corporation, Indianapolis, IN, USA). Then, 7 µl of the *Nde* I/*Mlu* I digested pJLin201 fragment and 1 µl of the digested pBM143b were mixed with 2 µl of 5X DNA dilution buffer (Roche Diagnostics Corporation, Indianapolis, IN, USA), 10 µl of 2X T4 DNA ligation buffer (Roche Diagnostics Corporation, Indianapolis, IN, USA), and 1 µl of T4 DNA ligase (Roche Diagnostics Corporation, Indianapolis, IN, USA) and incubated for 15 minutes at room temperature. Two microliters of the ligation were transformed into XL1-Blue Subcloning-Grade Competent Cells (Stratagene, La Jolla, CA, USA) cells and spread onto 2XYT plus ampicillin plates. Plasmid DNA was purified from several transformants using a BIOROBOT® 9600 and analyzed by DNA sequencing using a 3130XL Genetic Analyzer to identify a plasmid containing the desired *A. nidulans pyrG* insert. One plasmid with the expected DNA sequence was designated pBM175b.

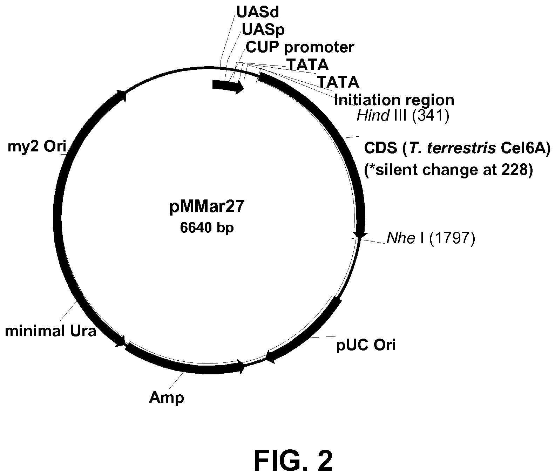

Plasmid pMMar27 was constructed from pBM175b and an amplified gene of *T. terrestris* Cel6A cellobiohydrolase II with overhangs designed for insertion into digested pBM175b. Plasmid pBM175b containing the *Thermomyces lanuginosus* lipase variant gene under control of the *CUP* I promoter contains unique *Hind* III and *Nhe* I sites to remove the lipase gene. Plasmid pBM175 was digested with these restriction enzymes to remove the lipase gene. After digestion, the empty vector was isolated by 1.0% agarose gel electrophoresis using TBE buffer where an approximately 5,215 bp fragment was excised from the gel and extracted using a QIAQUICK® Gel Extraction Kit. The ligation reaction (20 µl) was composed of 1X IN-FUSION® Buffer (BD Biosciences, Palo Alto, CA, USA), 1X BSA (BD Biosciences, Palo Alto, CA, USA), 1 µl of IN-FUSION® enzyme (diluted 1:10) (BD Biosciences, Palo Alto, CA, USA), 99 ng of pBM175b digested with *Hind* III and *Nhe* I, and 36 ng of the purified *T. terrestris* Cel6A cellobiohydrolase II PCR product. The reaction was incubated at room temperature for 30 minutes. A2 µl volume of the IN-FUSION® reaction was transformed into *E. coli* XL10-GOLD® Ultracompetent Cells. Transformants were selected on LB plates supplemented with 100 µg of ampicillin per ml. A colony was picked that contained the *T. terrestris* Cel6A inserted into the pBM175b vector in place of the lipase gene, resulting in pMMar27 (Figure 2). The plasmid chosen contained a PCR error at position 228 from the start codon, TCT instead of TCC, but resulted in a silent change in the *T. terrestris* Cel6A cellobiohydrolase II.

### Example 3: Construction of pEvFz1 expression vector

Expression vector pEvFz1 was constructed by modifying pBM120a (U.S. Patent 8,263,824) to comprise the NA2/NA2-tpi promoter, *A. niger* amyloglucosidase terminator sequence (AMG terminator), and *Aspergillus nidulans* orotidine-5'-phosphate decarboxylase gene (*pyrG*) as a selectable marker.

Plasmid pEvFz1 was generated by cloning the *A. nidulans pyrG* gene from pAILo2 (WO 2004/099228) into pBM120a. Plasmids pBM120a and pAILo2 were digested with *Nsi* I overnight at 37°C. The resulting 4176 bp linearized pBM120a vector fragment and the 1479 bp *pyrG* gene insert from pAILo2 were each purified by 0.7% agarose gel electrophoresis using TAE buffer, excised from the gel, and extracted using a QIAQUICK® Gel Extraction Kit.

The 1479 bp *pyrG* gene insert was ligated to the *Nsi* I digested pBM120a fragment using a QUICK LIGATION™ Kit (New England Biolabs, Beverly, MA, USA). The ligation reaction was composed of 1X QUICK LIGATION™ Reaction Buffer (New England Biolabs, Beverly, MA, USA), 50 ng of *Nsi* I digested pBM120a vector, 54 ng of the 1479 bp *Nsi* I digested *pyrG* gene insert, and 1 µl of T4 DNA ligase in a total volume of 20 µl. The ligation mixture was incubated at 37°C for 15 minutes followed at 50°C for 15 minutes and then placed on ice.

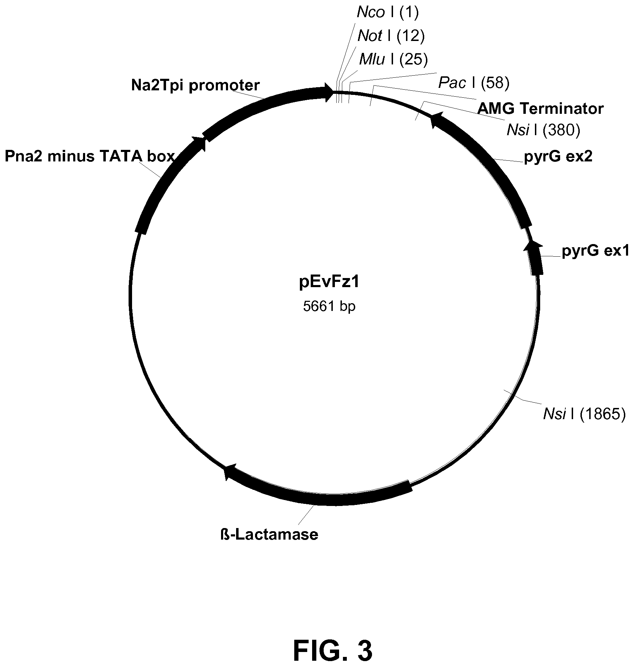

One µl of the ligation mixture was transformed into ONE SHOT@ TOP10 chemically competent *Escherichia coli* cells. Transformants were selected on 2XYT plus ampicillin plates. Plasmid DNA was purified from several transformants using a BIOROBOT® 9600 and analyzed by DNA sequencing using a 3130XL Genetic Analyzer to identify a plasmid containing the desired *A. nidulans pyrG* insert. One plasmid with the expected DNA sequence was designated pEvFz1 (Figure 3).

### Example 4: Construction of the plasmid pDLHD0006 as a yeast/E. colilA. oryzae shuttle vector

Plasmid pDLHD0006 was constructed as a base vector to enable *A. oryzae* expression cassette library building using yeast recombinational cloning. Plasmid pDLHD0006 was generated by combining three DNA fragments using yeast recombinational cloning: Fragment 1 containing the *E. coli* pUC origin of replication, *E. coli* beta-lactamase (*ampR*) selectable marker, URA3 yeast selectable marker, and yeast 2 micron origin of replication from pMMar27 (Example 2); Fragment 2 containing the 10 *amyR*/NA2-tpi promoter (a hybrid of the promoters from the genes encoding *Aspergillus niger* neutral alpha-amylase and *Aspergillus oryzae* triose phosphate isomerase and including 10 repeated binding sites for the *Aspergillus oryzae amyR* transcription factor), *Thermomyces lanuginosus* lipase open reading frame (ORF), and *Aspergillus niger* glucoamylase terminator from pJaL1262 (WO 2013/178674); and Fragment 3 containing the *Aspergillus nidulans pyrG* selection marker from pEvFz1 (Example 3).

| **pDLHD0006** | **PCR Contents** | **PCR Template** |
|---|---|---|
| Fragment 1 | *E. coli* ori/AmpR/URA/2 micron (4.1 kb) | pMMar27 |
| Fragment 2 | 10 amyR/NA2-tpi PR/lipase/Tamg (4.5 kb) | pJaL1262 |
| Fragment 3 | *pyrG* gene from pEvFz1 (1.7 kb) | pEvFz1 |

Fragment 1 was amplified using primers 613017 (sense) and 613018 (antisense) shown below. Primer 613017 was designed to contain a flanking region with sequence homology to Fragment 3 (lower case) and primer 613018 was designed to contain a flanking region with sequence homology to Fragment 2 (lower case) to enable yeast recombinational cloning between the three PCR fragments.
Primer 613017 (sense):
   ttaatcgccttgcagcacaCCGCTTCCTCGCTCACTGACTC (SEQ ID NO: 13)
613018 (antisense):
   acaataaccctgataaatgcGGAACAACACTCAACCCTATCTCGGTC (SEQ ID NO: 14)

Fragment 1 was amplified by PCR in a reaction composed of 10 ng of plasmid pMMar27, 0.5 µl of PHUSION® DNA Polymerase (New England Biolabs, Inc., Ipswich, MA, USA), 20 pmol of primer 613017, 20 pmol of primer 613018, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; and 30 cycles each at 98°C for 10 seconds, 60°C for 10 seconds, and 72°C for 1.5 minutes. The resulting 4.1 kb PCR product (Fragment 1) was used directly for yeast recombination with Fragments 2 and 3 below.

Fragment 2 was amplified using primers 613019 (sense) and 613020 (antisense) shown below. Primer 613019 was designed to contain a flanking region of sequence homology to Fragment 1 (lower case) and primer 613020 was designed to contain a flanking region of sequence homology to Fragment 3 (lower case) to enable yeast recombinational cloning between the three PCR fragments.
613019 (sense):
   agatagggttgagtgttgttccGCATTTATCAGGGTTATTGTCTCATGAGCGG (SEQ ID NO: 15)
613020 (antisense):
   ttctacacgaaggaaagagGAGGAGAGAGTTGAACCTGGACG (SEQ ID NO: 16)

Fragment 2 was amplified by PCR in a reaction composed of 10 ng of plasmid pJaL1262, 0.5 µl of PHUSION® DNA Polymerase, 20 pmol of primer 613019, 20 pmol of primer 613020, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; 30 cycles each at 98°C for 10 seconds, 60°C for 10 seconds, and 72°C for 2 minutes; and a 20°C hold. The resulting 4.5 kb PCR product (Fragment 2) was used directly for yeast recombination with Fragment 1 above and Fragment 3 below.

Fragment 3 was amplified using primers 613022 (sense) and 613021 (antisense) shown below. Primer 613021 was designed to contain a flanking region of sequence homology to Fragment 2 (lower case) and primer 613022 was designed to contain a flanking region of sequence homology to Fragment 1 (lower case) to enable yeast recombinational cloning between the three PCR fragments.
613022 (sense):
   aggttcaactctctcctcCTCTTTCCTTCGTGTAGAAGACCAGACAG (SEQ ID NO: 17)
613021 (antisense):
   tcagtgagcgaggaagcggTGTGCTGCAAGGCGATTAAGTTGG (SEQ ID NO: 18)

Fragment 3 was amplified by PCR in a reaction composed of 10 ng of plasmid pEvFz1 (Example 3), 0.5 µl of PHUSION® DNA Polymerase, 20 pmol of primer 613021, 20 pmol of primer 613022, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; 30 cycles each at 98°C for 10 seconds, 60°C for 10 seconds, and 72°C for 2 minutes; and a 20°C hold. The resulting 1.7 kb PCR product (Fragment 3) was used directly for yeast recombination with Fragments 1 and 2 above.

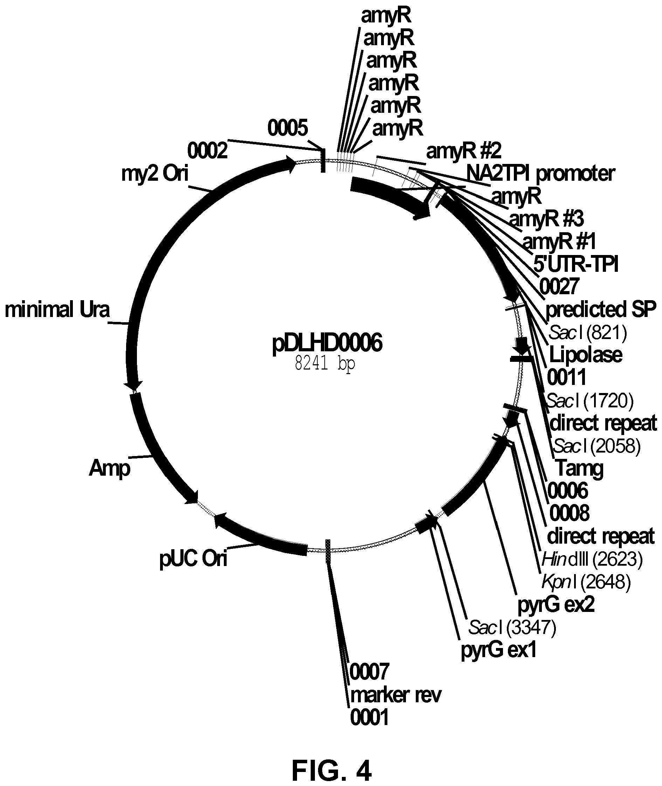

The following procedure was used to combine the three PCR fragments using yeast homology-based recombinational cloning. A 20 µl aliquot of each of the three PCR fragments was combined with 100 µg of single-stranded deoxyribonucleic acid from salmon testes (Sigma-Aldrich, St. Louis, MO, USA), 100 µl of competent yeast cells of strain YNG318 (*Saccharomyces cerevisiae* ATCC 208973), and 600 µl of PLATE Buffer (Sigma Aldrich, St. Louis, MO, USA), and mixed. The reaction was incubated at 30°C for 30 minutes with shaking at 200 rpm. The reaction was then continued at 42°C for 15 minutes with no shaking. The cells were pelleted by centrifugation at 5,000 x g for 1 minute and the supernatant was discarded. The cell pellet was suspended in 200 µl of autoclaved water and split over two agar plates containing Synthetic defined medium lacking uridine and incubated at 30°C for three days. The yeast colonies were isolated from the plate using 1 ml of autoclaved water. The cells were pelleted by centrifugation at 13,000 x g for 30 seconds and a 100 µl aliquot of glass beads were added to the tube. The cell and bead mixture was suspended in 250 µl of P1 buffer (QIAGEN Inc., Valencia, CA, USA) and then vortexed for 1 minute to lyse the cells. The plasmid DNA was purified using a QIAPREP® Spin Miniprep Kit. A 3 µl aliquot of the plasmid DNA was then transformed into *E. coli* ONE SHOT® TOP10 electrocompetent cells according the manufacturer's instructions. Fifty µl of transformed cells were spread onto LB plates supplemented with 100 µg of ampicillin per ml and incubated at 37°C overnight. Transformants were each picked into 3 ml of LB medium supplemented with 100 µg of ampicillin per ml and grown overnight at 37°C with shaking at 250 rpm. The plasmid DNA was purified from colonies using a QIAPREP® Spin Miniprep Kit. DNA sequencing with a 3130XL Genetic Analyzer was used to confirm the presence of each of the three fragments in a final plasmid designated pDLHD0006 (Figure 4).

### Example 5: Preparation of Arabidopsis thaliana xyloglucan endotransglycosylase 14

*Arabidopsis thaliana* xyloglucan endotransglycosylase (AtXET14; SEQ ID NO: 4 [native DNA sequence], SEQ ID NO: 5 [synthetic DNA sequence], and SEQ ID NO: 6 [deduced amino acid sequence]) was recombinantly produced in *Aspergillus oryzae* JaL355 (WO 2008/138835).

The vector pDLHD0039 was constructed to express the AtXET14 gene in multi-copy in *Aspergillus oryzae.* Plasmid pDLHD0039 was generated by combining two DNA fragments using restriction-free cloning: Fragment 1 containing the AtXET14 ORF and flanking sequences with homology to vector pDLHD0006 (Example 4), and Fragment 2 consisting of an inverse PCR amplicon of vector pDLHD0006.

Fragment 1 was amplified using primers AtXET14F (sense) and AtXET14R (antisense) shown below, which were designed to contain flanking regions of sequence homology to vector pDLHD0006 (lower case) for ligation-free cloning between the PCR fragments.
Primer AtXET14F (sense):
   ttcctcaatcctctatatacacaactggccATGGCCTGTTTCGCAACCAAACAG (SEQ ID NO: 19)
AtXET14R (antisense):
   agctcgctagagtcgacctaGAGTTTACATTCCTTGGGGAGACCCTG (SEQ ID NO: 20)

Fragment 1 was amplified by PCR in a reaction composed of 10 ng of a GENEART® vector pMA containing the AtXET14 synthetic DNA sequence cloned between the Sac I and *Kpn* I sites, 0.5 µl of PHUSION® DNA Polymerase (New England Biolabs, Inc., Ipswich, MA, USA), 20 pmol of primer AtXET14F, 20 pmol of primer AtXET14R, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; and 30 cycles each at 98°C for 10 seconds, 60°C for 10 seconds, and 72°C for 30 seconds. The resulting 0.9 kb PCR product (Fragment 1) was treated with 1 µl of *Dpn* I to remove plasmid template DNA. The *Dpn* I was added directly to the PCR tube, mixed well, and incubated at 37°C for 60 minutes, and then column-purified using a MINELUTE® PCR Purification Kit.

Fragment 2 was amplified using primers 614604 (sense) and 613247 (antisense) shown below.
614604 (sense):
   taggtcgactctagcgagctcgagatc (SEQ ID NO: 21)
613247 (antisense):
   catggccagttgtgtatatagaggattgaggaaggaagag (SEQ ID NO: 22)

Fragment 2 was amplified by PCR in a reaction composed of 10 ng of plasmid pDLHD0006, 0.5 µl of PHUSION® DNA Polymerase, 20 pmol of primer 614604, 20 pmol of primer 613247, 1 µl of 10 mM dNTPs, 10 µl of 5X PHUSION® HF buffer, and 35.5 µl of water. The reaction was incubated in an EPPENDORF® MASTERCYCLER® programmed for 1 cycle at 98°C for 30 seconds; and 30 cycles each at 98°C for 10 seconds, 60°C for 10 seconds, and 72°C for 4 minutes. The resulting 7.3 kb PCR product (Fragment 2) was treated with 1 µl of *Dpn* I to remove plasmid template DNA. *Dpn* I was added directly to the PCR tube, mixed well, and incubated at 37°C for 60 minutes, and then column-purified using a MINELUTE® PCR Purification Kit.

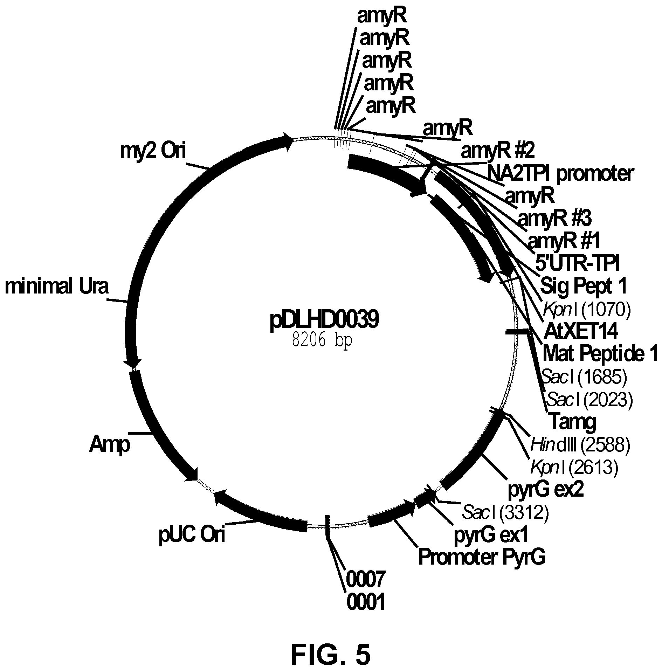

The two PCR fragments were combined using a GENEART® Seamless Cloning and Assembly Kit (Invitrogen, Carlsbad, CA, USA) according to manufacturer's instructions. Three µl of the resulting reaction product DNA were then transformed into *E. coli* ONE SHOT@ TOP10 electrocompetent cells. Fifty µl of transformed cells were spread onto LB plates supplemented with 100 µg of ampicillin per ml and incubated at 37°C overnight. Individual transformant colonies were picked into 3 ml of LB medium supplemented with 100 µg of ampicillin per ml and grown overnight at 37°C with shaking at 250 rpm. The plasmid DNA was purified from colonies using a QIAPREP® Spin Miniprep Kit according to the manufacturer's instructions. DNA sequencing with a 3130XL Genetic Analyzer was used to confirm the presence of each of both fragments in the final plasmid pDLHD0039 (Figure 5).

*Aspergillus oryzae* strain JaL355 was transformed with plasmid pDLHD0039 comprising the AtXET14 gene according to the following protocol. Approximately 2-5 x 10⁷ spores of *Aspergillus oryzae* JaL355 were inoculated into 100 ml of YP + 2% glucose + 10 mM uridine in a 500 ml shake flask and incubated at 28°C and 110 rpm overnight. Ten ml of the overnight culture was filtered in a 125 ml sterile vacuum filter, and the mycelia were washed twice with 50 ml of 0.7 M KCI-20 mM CaCl₂. The remaining liquid was removed by vacuum filtration, leaving the mat on the filter. Mycelia were resuspended in 10 ml of 0.7 M KCI-20 mM CaCl₂ and transferred to a sterile 125 ml shake flask containing 20 mg of GLUCANEX® 200 G per ml and 0.2 mg of chitinase per ml in 10 ml of 0.7 M KCI-20 mM CaCl₂. The mixture was incubated at 37°C and 100 rpm for 30-90 minutes until protoplasts were generated from the mycelia. The protoplast mixture was filtered through a sterile funnel lined with MIRACLOTH® into a sterile 50 ml plastic centrifuge tube to remove mycelial debris. The debris in the MIRACLOTH® was washed thoroughly with 0.7 M KCI-20 mM CaCl_{2,} and centrifuged at 2500 rpm (537 x *g*) for 10 minutes at 20-23°C. The supernatant was removed and the protoplast pellet was resuspended in 20 ml of 1 M sorbitol-10 mM Tris-HCI (pH 6.5)-10 mM CaCl₂. This step was repeated twice, and the final protoplast pellet was resuspended in 1 M sorbitol-10 mM Tris-HCI (pH 6.5)-10 mM CaCl₂ to obtain a final protoplast concentration of 2 x 10⁷/ml.

Two micrograms of pDLHD0039 were added to the bottom of a sterile 2 ml plastic centrifuge tube. Then 100 µl of protoplasts were added to the tube followed by 300 µl of 60% PEG-4000 in 10 mM Tris-HCI (pH 6.5)-10 mM CaCl₂. The tube was mixed gently by hand and incubated at 37°C for 30 minutes. Two ml of 1 M sorbitol-10 mM Tris-HCI (pH 6.5)-10 mM CaCl₂ were added to each transformation and the mixture was transferred onto 150 mm Minimal medium agar plates. Transformation plates were incubated at 34°C until colonies appeared.

Thirty-five transformant colonies were picked to fresh Minimal medium agar plates and cultivated at 34°C for four days until the strains sporulated. Fresh spores were transferred to 48-well deep-well plates containing 2 ml of YP + 2% maltodextrin, covered with a breathable seal, and grown for 4 days at 28°C with no shaking. After 4 days growth the culture medium was assayed for xyloglucan endotransglycosylase activity and for xyloglucan endotransglycosylase expression by SDS-PAGE.

Xyloglucan endotransglycosylase activity was measured using the iodine stain assay described in Example 1. The assay demonstrated the presence of xyloglucan endotransglycosylase activity in several transformants.

SDS-PAGE was performed as described in Example 1. SDS-PAGE analysis indicated that several transformants expressed a protein of approximately 32 kDa corresponding to AtXET14.

### Example 6: Generation of fluorescein isothiocyanate-labeled xyloglucan

Fluorescein isothiocyanate-labeled xyloglucan oligomers (FITC-XGOs) were generated by reductive amination of the reducing ends of xyloglucan oligomers (XGOs) according to the procedure described by Zhou et al., 2006, Biocatalysis and Biotransformation 24: 107-120), followed by conjugation of the amino groups of the XGOs to fluorescein isothiocyanate isomer I (Sigma Aldrich, St. Louis, MO, USA) in 100 mM sodium bicarbonate pH 9.0 for 24 hours at room temperature. Conjugation reaction products were concentrated to dryness *in vacuo,* dissolved in 0.5 ml of deionized water, and purified by silica gel chromatography, eluting with a gradient from 100:0:0.04 to 70:30:1 acetonitrile:water:acetic acid as mobile phase. Purity and product identity were confirmed by evaporating the buffer, dissolving in D₂O (Sigma Aldrich, St. Louis, MO, USA), and analysis by ¹H NMR using a Varian 400 MHz MercuryVx (Agilent, Santa Clara, CA, USA). Dried FITC-XGOs were stored at -20°C in the dark, and were desiccated during thaw.

Twenty-four ml of 10 mg of tamarind seed xyloglucan (Megazyme, Bray, UK) per ml of deionized water, 217 µl of 7.9 mg of FITC-XGOs per ml of deionized water, 1.2 ml of 400 mM sodium citrate pH 5.5, and 600 µl of 1.4 mg of VaXET16 per ml of 20 mM sodium citrate pH 5.5 were mixed thoroughly and incubated overnight at room temperature. Following overnight incubation, FITC-XG was precipitated by addition of ice cold ethanol to a final volume of 110 ml, mixed thoroughly, and incubated at 4°C overnight. The precipitated FITC-XG was washed with water and then transferred to Erlenmeyer bulbs. Residual water and ethanol were removed by evaporation using an EZ-2 Elite evaporator (SP Scientific/Genevac, Stone Ridge, NY, USA) for 4 hours. Dried samples were dissolved in water, and the volume was adjusted to 48 ml with deionized water to generate a final FITC-XG concentration of 5 mg per ml with an expected average molecular weight of 100 kDa.

### Example 7: Fluorescence polarization assay for xyloglucan endotransglycosylation activity

Xyloglucan endotransglycosylation activity was assessed using the following assay. Reactions of 200 µl containing 1 mg of tamarind seed xyloglucan per ml, 0.01 mg/ml FITC-XGOs prepared as described in Example 6, and 10 µl of appropriately diluted XET were incubated for 10 minutes at 25°C in 20 mM sodium citrate pH 5.5 in opaque 96-well microtiter plates. Fluorescence polarization was monitored continuously over this time period, using a SPECTRAMAX® M5 microplate reader (Molecular Devices, Sunnyvale, CA, USA) in top-read orientation with an excitation wavelength of 490 nm, an emission wavelength of 520 nm, a 495 cutoff filter in the excitation path, high precision (100 reads), and medium photomultiplier tube sensitivity. XET-dependent incorporation of fluorescent XGOs into non-fluorescent xyloglucan (XG) results in increasing fluorescence polarization over time. The slope of the linear regions of the polarization time progress curves was used to determine the activity.

### Example 8: Purification of Vigna angularis xyloglucan endotransglycosylase 16

One liter solutions of crude fermentation broth of *Vigna angularis* were filtered using a 0.22 µm STERICUP® filter (Millipore, Bedford, MA, USA) and the filtrates were stored at 4°C. Cell debris was resuspended in 1 liter of 0.25% TRITON® X-100 (4-(1,1,3,3-tetramethylbutyl)phenyl-polyethylene glycol; Sigma Aldrich, St. Louis, MO, USA)-20 mM sodium citrate pH 5.5, incubated at least 30 minutes at room temperature, and then filtered using a 0.22 µm STERICUP® filter. The filtrates containing *Vigna angularis* xyloglucan endotransglycosylase 16 (VaXET16) were pooled and concentrated to a volume between 500 and 1500 ml using a VIVAFLOW® 200 tangential flow concentrator (Millipore, Bedford, MA, USA) equipped with a 10 kDa molecular weight cutoff membrane.

The concentrated filtrates were loaded onto a 150 ml Q SEPHAROSE® Big Beads column (GE Healthcare Lifesciences, Piscataway, NJ, USA), pre-equilibrated with 20 mM sodium citrate pH 5.5, and eluted isocratically with the same buffer. The eluent was loaded onto a 75 ml Phenyl SEPHAROSE® HP column (GE Healthcare Lifesciences, Piscataway, NJ, USA) pre-equilibrated in 20% ethylene glycol-20 mM sodium citrate pH 5.5. VaXET16 was eluted using a linear gradient from 20% to 50% of 70% ethylene glycol in 20 mM sodium citrate pH 5.5 over 4 column volumes.

Purified VaXET16 was quantified using a BCA assay (Pierce, Rockford, IL, USA) in a 96-well plate format with bovine serum albumin (Pierce, Rockford, IL, USA) as a protein standard at concentrations between 0 and 2 mg/ml and was determined to be 1.40 mg/ml. VaXET16 homogeneity was confirmed by the presence of a single band at approximately 32 kDa using a 8-16% gradient CRITERION® Stain Free SDS-PAGE gel, and imaging the gel with a Stain Free Imager using the following settings: 5-minute activation, automatic imaging exposure (intense bands), highlight saturated pixels = ON, color = Coomassie, and band detection, molecular weight analysis and reporting disabled.

The activity of the purified VaXET16 was determined by measuring the rate of incorporation of fluorescein isothiocyanate-labeled xyloglucan oligomers into tamarind seed xyloglucan (Megazyme, Bray, UK) by fluorescence polarization (as described in Example 7). The apparent activity was 18.5 ± 1.2 P s⁻¹ mg⁻¹.

The purified VaXET16 preparation was tested for background enzyme activities including xylanase, amylase, cellulase, beta-glucosidase, protease, amyloglucosidase, and lipase using standard assays as shown below.

Xylanase activity was assayed using wheat arabinoxylan as substrate at pH 6.0 and 50°C. Xylan hydrolysis was assessed colorimetrically at 405 nm by addition of alkaline solution containing PHBAH.. One FXU(S) is defined as the endoxylanase activity using Shearzyme® (Novozymes A/S) as a standard.

Amylase activity was assayed using starch as substrate at pH 2.5 and 37°C. Starch hydrolysis was assessed by measuring the residual starch colorimetrically at 600 nm by addition of iodine solution.. One FAU(A) is defined as the acid alpha-amylase activity using acid fungal alpha-amylase (available from Novozymes A/S) as a standard.

Amylase activity was assayed using (4,6-ethylidene(G7)-*p*-nitrophenyl(G1)-α,D-maltoheptaoside (4,6-ethylidene-G7-pNP) as substrate at pH 7 and 37°C. Hydrolysis of the substrate produces p-nitrophenol, and was assessed colorimetrically at 405 nm. One FAU(F) is defined as fungal alpha-amylase units using Fungamyl® (Novozymes A/S) as a standard.

Cellulase activity was assayed using carboxymethylcellulose (CMC) as substrate at pH 5.0 and 50°C. CMC hydrolysis was assessed colorimetrically at 405 nm by addition of an alkaline solution containing para-hydroxybenzoic acid hydrazide (PHBAH). One CNU(B) is defined as the cellulase activity using NS22084 enzyme (Novozymes A/S) as a standard.

Beta-glucosidase activity was assayed using cellobiose as substrate at pH 5.0 and 50°C. Production of glucose from cellobiose was assessed using a coupled enzyme assay with hexokinase and glucose-6-phosphate dehydrogenase converting glucose to 6-phosphoglucanate following reduction of NAD to NADH at 340 nm.. One CBU(B) is defined as the amount of enzyme which releases 2 µmole of glucose per minute using cellobiase as a standard.

The protease assay was performed using an EnzChek® Protease Assay Kit (green fluorescence) (Life Technologies, Inc., Grand Island, NY, USA) with casein as substrate at pH 6 or 9 and ambient temperature. One KMTU is defined as a kilo microbial trypsin unit related to the amount of enzyme that produces 1 µmole of p-nitroaniline per minute.

Amyloglucosidase activity was assayed using maltose as substrate at pH 4.3 and 37°C.. Conversion of maltose to glucose was assessed using a coupled enzyme assay with hexokinase and glucose-6-phosphate dehydrogenase converting glucose to 6-phosphoglucanate following reduction of NAD to NADH at 340 nm. One AGU is defined as amyloglucosidase units using AMG® (Novozymes A/S) as a standard.

The 4-methylumbelliferyl beta-D-lactoside (MUL) assay was performed at pH 7 and ambient temperature and measured fluorometrically at 360 nm excitation and 465 nm emission.

Lipase activity was assayed using 4-nitropenyl butyrate (pNP-butyrate) as substrate at pH 7.5 and ambient temperature. pNP-butyrate hydrolysis was assessed colorimetrically following p-nitrophenol release at 405 nm. One LU is defined as the amount of enzyme which releases 1 µmole of titratable butyric acid using LIPOLASE® (Novozymes A/S) as a standard.

| **Assay** | **Substrate** | **Additional Assay Dilution** | **Activity Units** | **Activity Units/ml** |
|---|---|---|---|---|
| Xylanase FXU(S) | Wheat arabinoxylan | 4-fold | FXU(S) | ND |
| Amylase FAU(A) | Starch | 4-fold | FAU(A) | ND |
| Amylase FAU(F) | Ethylidene-G7-pNp | 4-fold | FAU(F) | ND |
| Cellulase CNU(B) | CMC | 4-fold | CNU(B) | ND |
| Beta-glucosidase CBU(B) | Cellobiose | 4-fold | CBU(B) | ND |
| Protease, pH 6 (EnzCheck) | Casein | none | KMTU | 740 |
| Protease, pH 9 (EnzCheck) | Casein | none | KMTU | 332 |
| Amyloglucosidase AGU | Maltose | 4-fold | AGU | ND |
| MUL | MUL | none | Unitless | ND |
| Lipase | pNP-Butyrate | none | LU | 0.02 |

### Example 9: Purification of Arabidopsis thaliana xyloglucan endotransglycosylase 14

The purification and quantification of the *Arabidopsis thaliana* xyloglucan endotransglycosylase 14 (AtXET14) was performed as described for VaXET16 in Example 8, except that elution from the Phenyl SEPHAROSE® HP column was performed using a linear gradient from 40% to 90% of 70% ethylene glycol in 20 mM sodium citrate pH 5.5 over 4 column volumes.

AtXET14 homogeneity was confirmed by the presence of a single band at approximately 32 kDa using a 8-16% CRITERION® Stain Free SDS-PAGE gel, and imaging the gel with a Stain Free Imager using the following settings: 5-minute activation, automatic imaging exposure (intense bands), highlight saturated pixels = ON, color = Coomassie, and band detection, molecular weight analysis and reporting disabled.

Purified AtXET14 was quantified using a BCA assay in a 96-well plate format with bovine serum albumin as a protein standard at concentrations between 0 and 2 mg/ml and was determined to be 1.49 mg/ml.

The activity of the purified AtXET14 was determined as described in Example 7. The apparent activity was 34.7 ± 0.9 P s⁻¹mg⁻¹.

The purified AtXET14 preparation was tested for background activities including xylanase, amylase, cellulase, beta-glucosidase, protease, amyloglucosidase, and lipase using standard assays as shown below. The standard assays are described in Example 8.

| **Assay** | **Substrate** | **Additional Assay Dilution** | **Activity Units** | **Activity Units/ml** |
|---|---|---|---|---|
| Xylanase FXU(S) | Wheat arabinoxylan | 4-fold | FXU(S) | ND |
| Amylase FAU(A) | Starch | 4-fold | FAU(A) | ND |
| Amylase FAU(F) | Ethylidene-G7-pNp | 4-fold | FAU(F) | ND |
| Cellulase CNU(B) | CMC | 4-fold | CNU(B) | ND |
| Beta-glucosidase CBU(B) | Cellobiose | 4-fold | CBU(B) | ND |
| Protease, pH 6 (EnzCheck) | Casein | none | KMTU | 82 |
| Protease, pH 9 (EnzCheck) | Casein | none | KMTU | 53 |
| Amyloglucosidase AGU | Maltose | 4-fold | AGU | ND |
| MUL | MUL | none | Unitless | ND |
| Lipase | pNP-Butyrate | none | LU | 0.24 |

### Example 10: Effect of Vigna angularis xyloglucan endotransglycosylase 16 modified kaolin on filler retention in handsheet compositions

A 0.3% (w/w) slurry of bleached eucalyptus kraft fiber (BEKP) was prepared with tap water. To prepare a single hand sheet, 800 ml of the slurry, containing 2.4 oven dry grams of fiber, were transferred to a 1 liter plastic beaker. Aliquots of water (control) or kaolin slurry were added to the blender. The kaolin slurries were generated by suspending 2 g of kaolin (Sigma Aldrich, St. Louis, MO, USA) in 50 ml of deionized water, or in 50 ml of 20 mM citrate pH 5.5, or in 50 ml of 20 mM citrate pH 5.5 containing 125 mg of tamarind kernel powder xyloglucan with or without 1 µM VaXET16. Unmodified and modified kaolin slurries were dosed to deliver a 10% equivalent weight with the fiber in the sample (*i.e.,* 0.24 oven dry grams of kaolin per sample). The samples were then mixed for 30 seconds by an impeller at low speed. Immediately after mixing, each sample was transferred to the half-full deckle of a standard hand sheet former. The deckle was then completely filled, agitated, and drained to form a sheet. Each sheet was couched, pressed, and dried according to TAPPI standard procedure T-205 sp-95 "Forming Handsheets for Physical Testing of Pulp". Eight sheets were prepared from each of the trial sets listed in Table 1.

Three sheets from each set were used to determine ash content according to TAPPI standard T-211 om-02 "Ash in wood, pulp, paper and paperboard: combustion at 525°C". The physical strength properties of the remaining five sheets from each set were determined according to TAPPI standard T-220 sp-96 "Physical Testing of Pulp Handsheets".

**Table 1: Trial description**

| Trial | Fiber type | Total fiber (odg) | Kaolin/Fiber % (w/w) | Clay description | # of sheets |
|---|---|---|---|---|---|
| 1 | BEKP | 2.4 | 0 | None | 8 |
| 2 | BEKP | 2.4 | 10 | Kaolin in water | 8 |
| 3 | BEKP | 2.4 | 10 | Kaolin in citrate buffer | 8 |
| 4 | BEKP | 2.4 | 10 | Kaolin + XG | 8 |
| 5 | BEKP | 2.4 | 10 | Kaolin + XG + VaXET16 | 8 |
| 6 | BEKP | 2.4 | 10 | Kaolin + VaXET16 | 8 |

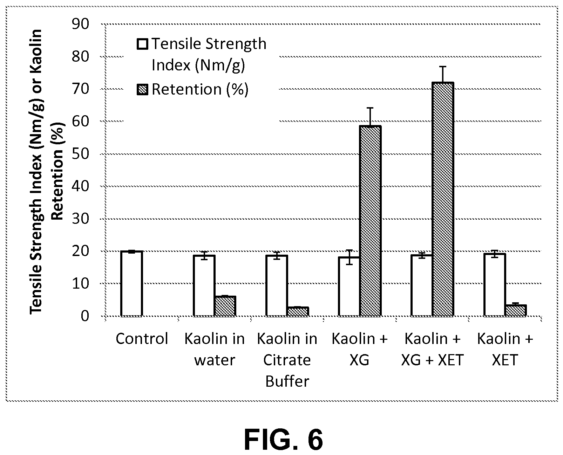

The filler retention results are presented in Figure 6. Although interactions between kaolin and xyloglucan resulted in significant retention of the filler in the forming web of BEKP, modification of the xylogucan by VaXET16 in the presence of kaolin produced a material with even greater retention. The results suggest that modification of kaolin in the presence of XET and xyloglucan produced filler with significantly improved retention in fibrous webs (*e.g.,* paper and board).

Increased retention of mineral fillers is desirable to reduce costs and impart specific optical and performance properties to paper and board. However, minerals obstruct the fiber-to-fiber bonding that is essential to build strength in the final product. Therefore, an upper limit of inclusion generally exists. Physical testing results indicate that, even with the significantly improved retention of kaolin, the strength properties of the handsheets were not significantly affected (Figure 6).

Table 2 lists the physical properties of the handsheets, tested as described above. For most of the properties, the change was small despite the large increase in kaolin retained in the handsheets when incubated with xyloglucan and VaXET16. These data indicate that xyloglucan and particularly xyloglucan with VaXET16 can be used to increase the retention of kaolin filler in paper without the use of flocculants or other retention aids, while maintaining the physical properties of the paper produced.

**Table 2. Physical testing and ash content data obtained from 120 g/m² handsheets prepared from bleached eucalyptus fiber in the absence or presence of various kaolin slurries.**

| Physical Test | Units | Control | Kaolin in water | Kaolin in citrate | Kaolin + XG | Kaolin + XG + XET | Kaolin + XET |
|---|---|---|---|---|---|---|---|
| Basis wt. | g/m² | 120.8 | 122.1 | 121.4 | 127.8 | 131.0 | 122.3 |
| Caliper | Mils | 0.05 | 0.05 | 0.05 | 0.05 | 0.05 | 0.05 |
| App. Density | g/cm³ | 2.5 | 2.6 | 2.6 | 2.6 | 2.6 | 2.6 |
| Dry Tensile | (Max load) kN/m | 36.0 | 34.0 | 33.8 | 34.7 | 36.7 | 35.1 |
| Bulk | cm³/g | 0.40 | 0.39 | 0.39 | 0.39 | 0.38 | 0.39 |
| Elongation | % | 2.6 | 2.5 | 2.6 | 2.4 | 2.7 | 2.7 |
| TEA† | J/m² | 49.6 | 43.4 | 43.7 | 41.2 | 50.1 | 46.5 |
| Tit | N·m/g | 19.9 | 18.6 | 18.6 | 18.1 | 18.7 | 19.1 |
| Burst Index | kPa·m²/g | 1.5 | 1.5 | 1.5 | 1.4 | 1.4 | 1.5 |
| Tear Index | mN·m²/g | 7.8 | 5.9 | 6.8 | 8.2 | 6.9 | 6.6 |
| Ash Content | % | 0 | 6.0 | 2.6 | 58.5 | 71.9 | 3.3 |

| | | | | | | | |
|---|---|---|---|---|---|---|---|
| Note: when applicable, kaolin was dosed at 10% (w/w) with the dry fiber. † TEA refers to Tensile Energy Absorption. ‡TI refers to Tensile Index. | | | | | | | |

### Example 11: Binding of fluorescein isothiocyanate-labeled xyloglucan to kaolin

To test and quantify kaolin-binding by xyloglucan, fluorescein isothiocyanate-labeled xyloglucan (FITC-XG) was used as a reporter and residual solution fluorescence was measured following incubation in either the presence or absence of kaolin. FITC-XG was generated as described in Example 6. *Vigna angularis* XET16 was purified as described in Example 8.

Binding reactions of 500 µl were performed in sealed 1.1 ml 96-deep well plates (Axygen, Union City, CA, USA). Kaolin (Sigma Aldrich, St. Louis, MO, USA) in an amount of 0 to 20 mg per ml was incubated with or without 1 µM VaXET16, and with or without 1 mg of FITC-XG per ml of 50 mM sodium citrate pH 5.5 at either 25°C or 37°C in an INNOVA® 40 shaker incubator (New Brunswick Scientific, Enfield, CT, USA) for up to 5 days. Plates were sealed with an ALPS 3000 plate sealer (Thermo Scientific, Waltham, MA, USA) and then wrapped in aluminum foil to preserve the fluorophore during incubation.

Following incubation for 1, 2, and 5 days, the deep well plates were centrifuged at 3000 rpm for 5 minutes using a LEGEND™ RT Plus centrifuge (Thermo Scientific, Waltham, MA, USA) to pellet the kaolin with any associated FITC-XG and fluorescence intensity of the supernatant was measured in the following manner. Aliquots of 200 µl of each supernatant were removed and transferred to a Costar 9017 flat bottomed microtiter plate (Corning, Tewksbury, MA, USA). Fluorescence intensity was measured using a SPECTRAMAX® M5 microplate reader in bottom read format with an excitation wavelength of 488 nm, an emission wavelength of 520 nm, and a cutoff filter of 495 nm, in high precision mode (100 reads) and medium photomultiplier tube sensitivity settings. Fluorescence spectra were measured using the same samples and excitation settings as described for intensity measurements, measuring emission at wavelengths from 500 to 625 nm.

After measuring the fluorescence intensity, each sample aliquot was returned to its original reaction. The plate was resealed, rewrapped in foil, and placed back in the incubator to continue the binding reaction.

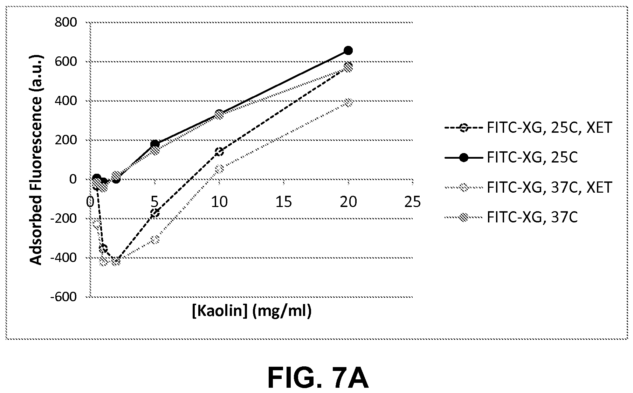

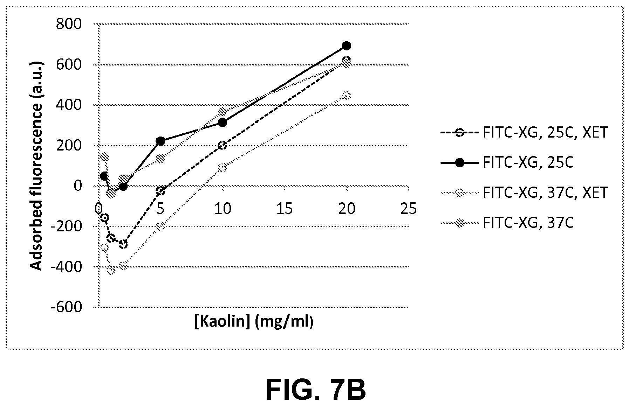

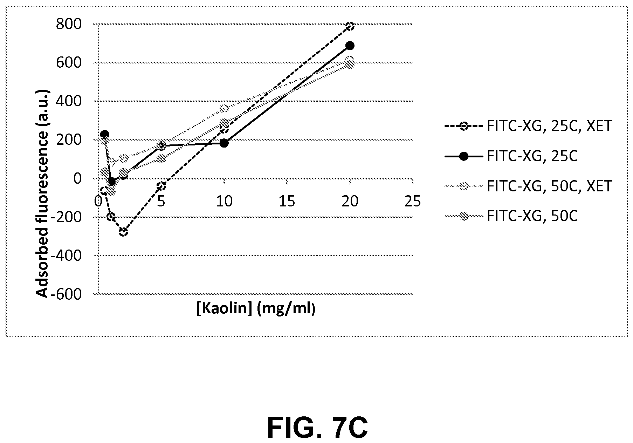

Figure 7 shows the increase of FITC-XG fluorescence adsorbed to kaolin with increasing mass of kaolin, relative to a control incubation performed without kaolin. Figure 7A shows kaolin titration after 1 day of incubation; Figure 7B shows kaolin titration after 2 days of incubation; and Figure 7C shows kaolin titration after 5 days of incubation. With increasing masses of kaolin, a decreasing fluorescence intensity in the supernatant phase and higher fluorescence adsorbed to the kaolin were observed. Similarly, in the presence of VaXET16, as the amount of kaolin in the reaction increased, the amount of fluorescence associated with the kaolin increased. At very low concentrations of kaolin, the solution phase fluorescence increased rather than decreased, yielding an apparent adsorbed fluorescence of less than zero.

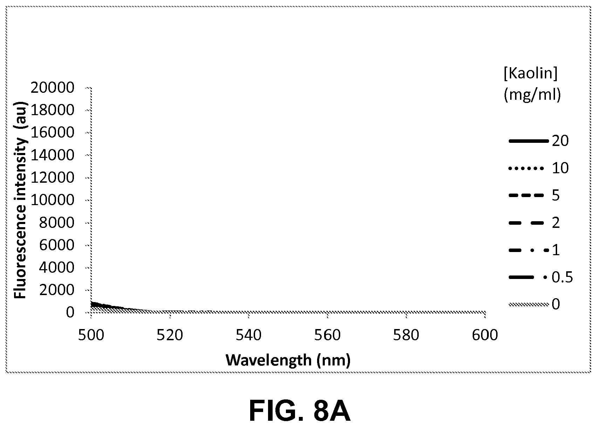

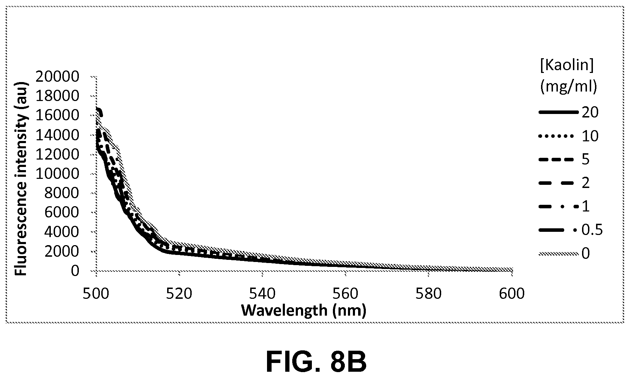

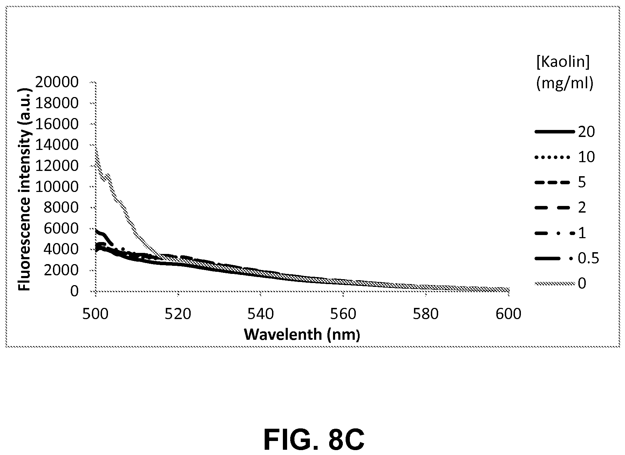

To confirm that fluorescence intensity did increase, fluorescence spectra were measured and are shown in Figure 8. Figure 8A shows the fluorescence spectra of supernatants of various kaolin concentrations incubated without FITC-XG. Figure 8B shows the fluorescence spectra of supernatants of various kaolin concentrations incubated with FITC-XG. Figure 8C shows the fluorescence spectra of supernatants of various concentrations of kaolin incubated with FITC-XG and VaXET16.

In the absence of VaXET16, high emission intensity was observed from 500 to 520 nm, which was attributed to scatter of the excitation radiation caused by aggregates of the xyloglucan. In the presence of VaXET16, when kaolin was absent a similar light scatter peak was observed. However, when kaolin was present the emission intensity between 500 and 520 nm was dramatically reduced, indicating no light scatter. These results indicate that the average particle size in solution was much smaller; thus the xyloglucan aggregates were dispersed by VaXET16 in the presence of kaolin. In both cases, the xyloglucan was bound to kaolin, functionalizing the kaolin with polysaccharide. When VaXET16 was present, the xyloglucan appeared more dispersed in the presence of kaolin than when VaXET16 was absent.

### Example 12: Binding of fluorescein isothiocyanate-labeled xyloglucan to kaolin by confocal microscopy

The reaction mixtures described in Example 11 were analyzed by laser scanning confocal microscopy according the following procedure. Aliquots of 300 µl of each reaction were removed, transferred to a 96-well, 0.45 micron PVDF filter plate (Millipore, Billerica, MA, USA), and centrifuged at 3000 rpm for 10 minutes using a LEGEND™ RT Plus centrifuge. The retentates were washed three times by resuspension in 300 µl of deionized water, mixed thoroughly, and then centrifuged as above. Washed kaolin retentates were then resuspended in 300 µl of deionized water and transferred to microcentrifuge tubes. Samples were stored at 4°C until analyzed. Approximately 20 µl of each sample were applied to a FisherFinest Premium 3"x1"x 1 mm microscope slide (Fisher Scientific, Inc., Pittsburg, PA, USA) and covered with a Fisherbrand 22x22-1.5 microscope coverslip (Fisher Scientific, Inc., Pittsburg, PA, USA) before sealing the coverslip to the slide using clear nail polish.

Fluorescence arising from FITC-XG associated with kaolin was imaged using an Olympus FV1000 laser scanning confocal microscope (Olympus, Center Valley, PA, USA) with a 10X air gap objective lens. Excitation was performed using the 488 nm line of the argon ion laser, and emission intensity was detected by integrating intensity from 500 to 520 nm incident on the photomultiplier tube detector through an emission monochromator. The photomultiplier (PMT) voltage settings were 678 with an offset setting of 3 for all images. Post scan image analysis was performed using FIJI (NIH, Bethesda, MD, USA) and MATLAB® (The Mathworks, Natick, MA, USA).

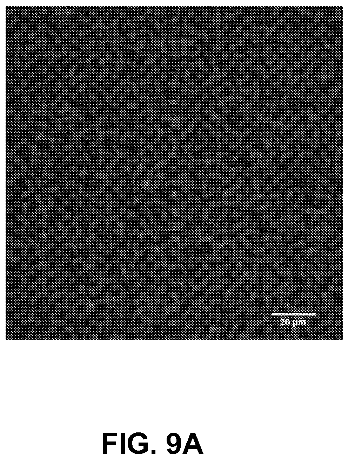

Figure 9A shows the confocal microscopy image of kaolin incubated with no FITC-XG overlaying the fluorescence emission with transmittance. From the image, no substantial fluorescence intensity was observed. The average pixel intensity was 56.69 ± 23.92.

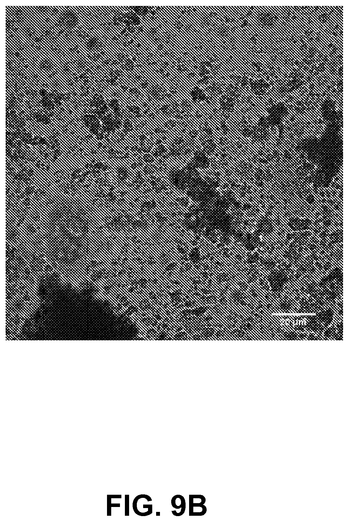

Figure 9B shows the confocal microscopy image of kaolin incubated with FITC-XG overlaying the fluorescence emission with transmittance. The average pixel intensity was 211.49 ± 159.37.

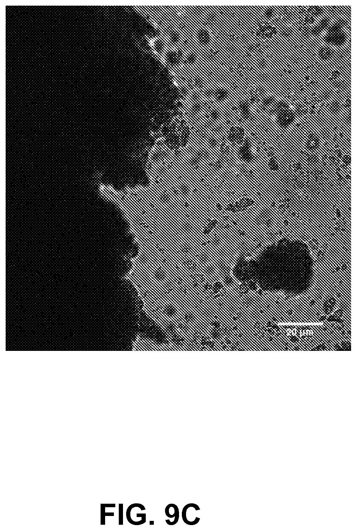

Figure 9C shows the confocal microscopy image of kaolin incubated with FITC-XG and VaXET16 overlaying the fluorescence emission with transmittance. The average pixel intensity was 185.26 ± 161.28.

Comparing the 3 images, clear differences were observed. Kaolin incubated without FITC-XG had a fluorescence intensity only slightly above background and significantly less fluorescence intensity than kaolin incubated with FITC-XG or FITC-XG and VaXET16. The rheology of the kaolin was also clearly different between samples incubated with and without FITC-XG. Kaolin was uniformly dispersed and appeared homogenous at this level of magnification when incubated without FITC-XG. Conversely, in the presence of FITC-XG or FITC-XG and VaXET16, the kaolin appeared to cluster or aggregate, and bright fluorescent spots were observed. Since the samples were extensively washed prior to microscopy, the fluorescent spots arose from FITC-XG bound to the kaolin, and these images indicate that FITC-xyloglucan had altered the rheology of kaolin.

### Example 13: Effect on physical properties of kaolin after incubation with xyloglucan or xyloglucan and Vigna angularis xyloglucan endotransglycosylase 16

In 100 ml glass bottles, 5 g of kaolin were incubated with or without 2.38 mg/ml tamarind seed xyloglucan and with or without 1.1 µM VaXET16 in 20 mM sodium citrate pH 5.5. The samples were mixed thoroughly, then placed horizontally in an INNOVA® 40 shaker incubator and incubated at 25°C overnight with shaking at 150 rpm. After incubation, the samples were mixed vigorously and then aliquoted into two 50 ml Centristar conical tubes (Corning, Tewksbury, MA, USA). The aliquots were centrifuged at 3200 rpm for 40 minutes using a LEGEND™ RT Plus centrifuge. The kaolin pellets were either resuspended and stored at 4°C or the supernatants were decanted and the kaolin pellets resuspended in approximately 50 ml of deionized water. The resuspended samples were incubated at 25°C overnight with shaking at 150 rpm, centrifuged, decanted, and resuspended in 50 ml of deionized water, and incubated overnight with shaking at 150 rpm two additional times, for a total of 3 washes.

Figure 10A shows photographs of the 50 ml conical tubes containing (1) kaolin, (2) kaolin incubated with xyloglucan, and (3) kaolin incubated with xyloglucan and VaXET16, following centrifugation. Kaolin treated with xyloglucan or xyloglucan and VaXET16 completely pelleted out during centrifugation while untreated kaolin remained partially suspended. These results indicate that the mass of the kaolin particles treated with xyloglucan increased or their density decreased; both are indications that xyloglucan associated with the kaolin, and possibly facilitated binding of kaolin particles together.

Figure 10B shows photographs of polystyrene serological pipets following contact with (1) kaolin, (2) kaolin incubated with xyloglucan, and (3) kaolin incubated with xyloglucan and VaXET16. Kaolin treated with xyloglucan or with xyloglucan and VaXET16 adhered to the polystyrene. These results indicate that xyloglucan facilitated binding of kaolin particles to plastic.

Figure 10C shows photographs of the 50 ml conical tubes containing (1) kaolin, (2) kaolin incubated with xyloglucan, and (3) kaolin incubated with xyloglucan and VaXET16, following extensive washing and resuspension in water. Kaolin treated with xyloglucan or with xyloglucan and VaXET16 and then extensively washed and centrifuged did not fully resuspend when applied to a vortex mixer. Large particles of kaolin appeared to clump or aggregate together and settled quickly. These results indicate that, even following extensive washing, xyloglucan remained bound to kaolin particles and when the particles were compressed by centrifugation, xyloglucan bound kaolin particles together. These observations are consistent with the confocal microscopy data described in Example 12 kaolin clustered or aggregated when incubated with xyloglucan or xyloglucan and VaXET16. Additionally, the data indicates that incubation of kaolin with xyloglucan or xyloglucan and VaXET16 increased the adhesion of kaolin to surfaces or other substances such as polystyrene.

### Example 14: Fluorescein isothiocyanate-labeled xyloglucan binding to titanium (IV) oxide

To test and quantify titanium (IV) oxide-binding by xyloglucan, FITC-XG was used as a reporter and residual solution fluorescence was measured following incubation in either the presence or absence of titanium (IV) oxide. FITC-XG was generated as described in Example 6. *Arabidopsis thaliana* XET14 was purified as described in Example 9. Binding was assessed as described in Example 11, with the following exceptions. A 10% slurry was generated by suspending 1 g of titanium (IV) oxide (mixture of rutile and anatase, particle size < 100 nm) (Sigma Aldrich, St. Louis, MO, USA) in 10 ml of 20 mM sodium citrate pH 5.5. The slurry was resuspended by inversion and 200 µl were pipetted to each binding reaction. TiO₂-binding reactions of 500 µl in 20 mM sodium citrate contained TiO₂ and either 1 mg/ml FITC-XG or 1 mg/ml FITC-XG with 1 µM AtXET14. Control reactions contained TiO₂ with no FITC-XG and AtXET14, or FITC-XG without TiO₂. Samples were mixed thoroughly with a pipet and then incubated under ambient conditions for 48 hours. At the indicated times, the 1.1 ml, 96-deep well plates were centrifuged at 3000 rpm (∼ 2200 x *g*) for 15 minutes, 100 µl aliquots of each of the supernatants were removed, and fluorescence intensity measured as described in Example 11. Aliquots were returned to their respective reaction well, wells were mixed thoroughly with a pipet, and plates were resealed.

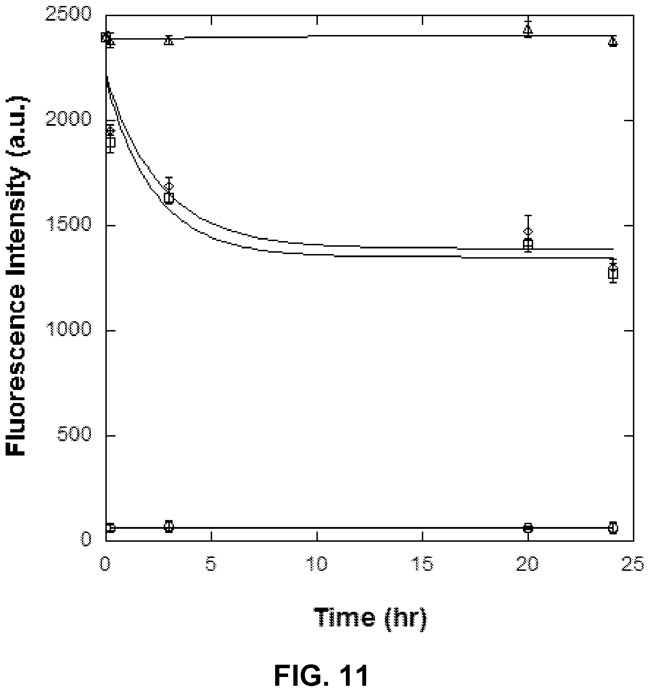

Figure 11 shows the fluorescence intensity of the supernatants of TiO₂-binding reactions and control incubations at various times. Open circles: TiO₂ with no FITC-XG; squares: TiO₂ with FITC-XG; diamonds: TiO₂ with FITC-XG and AtXET14; triangles: FITC-XG with no TiO₂. From the plot, it is evident that the fluorescence intensity of the supernatant of TiO₂ incubated with FITC-XG or FITC-XG and AtXET14 decreased sharply with time as the FITC-XG bound to TiO₂ and was removed from solution. Conversely, FITC-XG fluorescence did not decrease, and TiO₂ had a fluorescence intensity indistinguishable from the background. These data indicate that FITC-XG bound to TiO₂.

### Example 15: Xyloglucan and xyloglucan endotransglycosylase-mediated binding of silicon dioxide to multi-fiber fabrics

Binding of fluorescently-labeled silica (FITC-silicon oxide; Corpuscular Inc., Cold Spring, NY, USA) to multi-fiber fabric (Kimble Chase Life Science and Research Products LLC) was assessed using the following protocol. Multi-fiber fabric was cut into strips of approximately 1 centimeter width. In Costar #3524, 48-well cell culture plates (Corning, Tewksbury, MA, USA), the multi-fiber fabric strips were rolled and added to 1.5 ml solutions containing 20 mM phosphate buffer pH 7 with or without 2.5% (*w*/*v*) fluorescent silicon oxide, with or without 1.25 mg/ml tamarind seed xyloglucan (Megazyme, Ireland), with or without 6 µg/ml xyloglucan oligomers and with or without 0.3 µM VaXET16. The plates were wrapped in foil and incubated for 4 hours in a 25°C INNOVA® 40 shaker incubator with shaking at 220 rpm.

Following incubation, the multi-fiber fabric strips were removed from the wells and rinsed under deionized water for approximately 30 seconds. The multi-fiber fabric strips were then placed in new Costar #3524, 48-well cell culture plates and incubated with 1.5 ml of 20 mM phosphate pH 7 for 72 hours.

The multi-fiber fabric strips were analyzed for fluorescence using a Gel Doc™ EZ Imager (Bio-Rad Laboratories, Inc., Hercules, CA, USA) with Image Lab version 3.0 software (Bio-Rad Laboratories, Inc., Hercules, CA, USA) on SYBR® Green settings. The multi-fiber strips were exposed for 0.02 seconds and a TIFF image was obtained. Intensity histograms for each fabric were determined using ImageJ 1.47n software (National Institutes of Health, USA).

The multi-fiber fabric test strips were then washed in 10 ml of deionized water containing 20 µl of Tide® for sensitive skin (Procter and Gamble, USA) for 30 minutes at 37°C in 15 ml Corning® polypropylene centrifuge tubes (Corning, Tewksbury, MA, USA). After washing, the strips were rinsed extensively under deionized water. The strips were analyzed for fluorescence as described above.

In the presence of the FITC-SiO₂, fluorescence intensity was observed on the horizontal fibers of the test fabric incubated under each condition. Several fabrics additionally showed strong fluorescence when the SiO₂ was incubated with xyloglucan or particularly xyloglucan and VaXET16, including acrylic (Creslan), polyethylene terephthlate (Dacron 54 and 64), nylon 6.6, silk, viscose rayon and wool. The presence of xyloglucan oligomers did not facilitate the association, and the addition of xyloglucan oligomers to xyloglucan and VaXET16 reduced the amount of association, potentially by reducing the degree of polymerization of xyloglucan in a VaXET16-dependent manner.

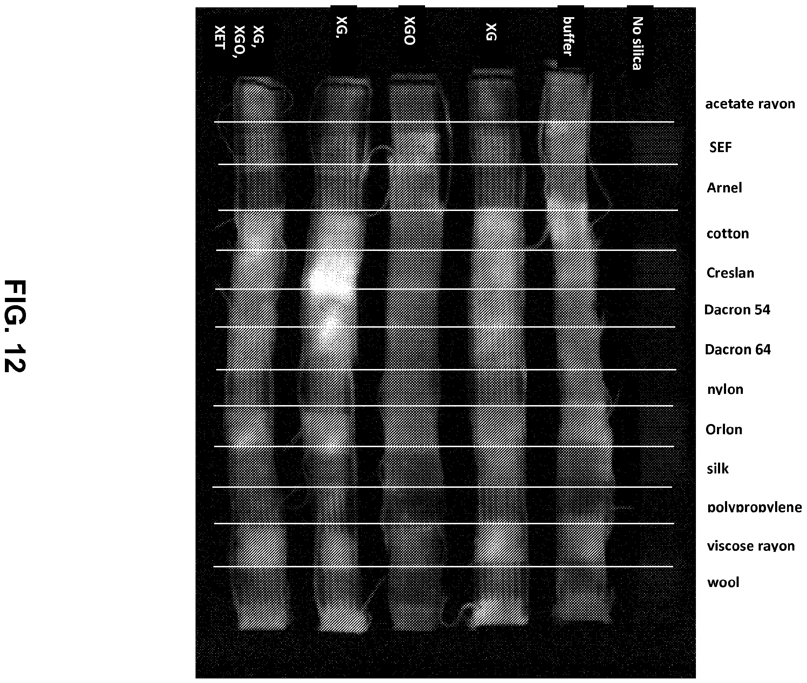

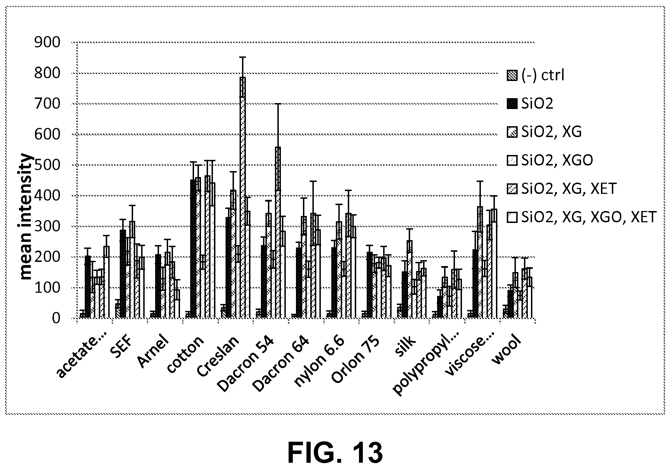

Figure 12 shows the fluorescence image of the variously treated test fabrics following additional washing with a standard laundry detergent; the treatments and the component fabrics are indicated. Figure 13 shows the mean fluorescence intensity of each fabric treatment following washing in detergent. The amount of SiO₂ fluorescence associated with the fabric was enhanced by xyloglucan or xyloglucan and VaXET16 for cotton, acrylic (Creslan), polyethylene terephthalate (Dacron 54 and 64), nylon 6.6, acrylic (Orlon 75), silk, polypropylene, viscose rayon, and wool. Acrylic and polyethylene terephthalate showed the largest VaXET16-dependent enhancement of the association. These data indicate that xyloglucan and particularly xyloglucan with VaXET16 can functionalize a variety of non-cellulosic fabrics with SiO₂.

The inventions described and claimed herein are not to be limited in scope by the specific aspects herein disclosed, since these aspects are intended as illustrations of several aspects of the invention. Any equivalent aspects are intended to be within the scope of the inventions. Indeed, various modifications of the inventions in addition to those shown and described herein will become apparent to those skilled in the art from the foregoing description. Such modifications are also intended to fall within the scope of the appended claims. In the case of conflict, the present disclosure including definitions will control.

## Claims

1. A method for linking materials comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; and (h) a composition comprising a xyloglucan endotransglycosylase and a xyloglucan oligomer, under conditions leading to a modified material comprising polymeric xyloglucan functionalized with a chemical group, under conditions leading to linking between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan:xyloglucan xyloglucanotransferase (EC 2.4.1.207).

2. A method for producing a composite material comprising treating two or more materials with a composition selected from the group consisting of (a) a composition comprising a xyloglucan endotransglycosylase, a polymeric xyloglucan, and a functionalized xyloglucan oligomer comprising a chemical group; (b) a composition comprising a xyloglucan endotransglycosylase, a polymeric xyloglucan functionalized with a chemical group, and a functionalized xyloglucan oligomer comprising a chemical group; (c) a composition comprising a xyloglucan endotransglycosylase, a polymeric xyloglucan functionalized with a chemical group, and a xyloglucan oligomer; (d) a composition comprising a xyloglucan endotransglycosylase, a polymeric xyloglucan, and a xyloglucan oligomer; (e) a composition comprising a xyloglucan endotransglycosylase and a polymeric xyloglucan functionalized with a chemical group; (f) a composition comprising a xyloglucan endotransglycosylase and a polymeric xyloglucan; (g) a composition comprising a xyloglucan endotransglycosylase and a functionalized xyloglucan oligomer comprising a chemical group; and (h) a composition comprising a xyloglucan endotransglycosylase and a xyloglucan oligomer, under conditions leading to a modified material comprising polymeric xyloglucan functionalized with a chemical group, under conditions leading to association between the two or more materials, wherein the linking between the two or more materials occurs via xyloglucan or xyloglucan derivatives forming covalent or non-covalent associations with the two or more materials, and wherein the xyloglucan endotransglycosidase is a xyloglucan :xyloglucan xyloglucanotransferase (EC 2.4.1.207).

3. The method of claim 1 or 2, wherein the material(s) is selected from the group consisting of a cellulose, a cellulosic material, a lignocellulosic material, a modified cellulosic material, a cellulose derivative material, a non-cellulosic natural polymer, a synthetic polymer, and a mineral.

4. The method of claim 1 or 2, wherein the material(s) is selected from the group consisting of paper, board, pulp, cotton, wool, yarn, jute, cellulosic biomass, lignocellulosic biomass, filter paper, cellulose acetate, carboxymethylcellulose, rayon, viscose, nitrocellulose lacquer, wood, wood veneer, laminated veneer lumber (LVL), wood laminates, cardboard, oriented strand board (OSB), chipboard, and plywood.

5. The method of claims 1-4, wherein the polymeric xyloglucan is functionalized prior to treating the material, while treating the material, or after treating the material.

6. The method of claims 1, wherein the functionalized material is subsequently unfunctionalized by addition of xyloglucanase, endo-β-1-4 glucanase, cellulase, or combinations thereof.

7. The method of claim 6, wherein the subsequently unfunctionalized material is refunctionalized by addition of polymeric xyloglucan functionalized with a chemical group or functionalized xyloglucan oligomers and xyloglucan endotransglycosylase.

8. The method of claim 1-5, wherein the linked or composite materials are linked stepwise, by association of one or more materials with xyloglucan and/or xyloglucan endotransglycosylase, followed by subsequent addition of additional materials with or without xyloglucan and with or without xyloglucan endotransglycosylase.

9. The method of claim 1 or 2, wherein the linked or composite materials are subsequently unlinked by addition of xyloglucanase, endo-β-1-4 glucanase, cellulase, or combinations thereof.

10. The method of claims 9, wherein the subsequently unlinked materials are relinked by addition of xyloglucan or polymeric xyloglucan functionalized with a chemical group and xyloglucan endotransglycosylase.

11. The method of any of claims 1-10, wherein the linked or composite material comprise one or more improved properties, wherein the one or more improvement is (are) selected from the group consisting of resistance to rot, decay, senescence, oxidation, flame or chemical resistance, UV-resistance, enhanced color properties improved dye or color uptake or retention, specific affinities, specific chemical reactivity, fungicidal or fungistatic properties, herbicidal or herbistatic properties, bacteriocidal or bacteriostatic properties, microbiocidal or microbiostatic properties non-specific affinities, chemical reactivity or affinity towards or resistance to specific chemicals, chemical groups, toxins, metals, salts, ions, polypeptides or desired analytes, including biological, radiological, chemical, etc. enhanced water-repellency, weather resistance, enhanced tensile strength, tear resistance, altered or improved optical properties, conductive properties, thermal properties, enzymatic activity, blending or association properties, anti-wrinkling, anti-piling, anti-shrink, surface properties, hydrophobicity, hydrophilicity, feel, texture, or hand feel properties, magnetic properties, rheology, and compositional properties.

12. The method of any of claims 1-11, wherein the average molecular weight of the polymeric xyloglucan or the polymeric xyloglucan functionalized with a chemical group ranges from 2 kDa to about 500 kDa.

13. The method of any of claims 1-12, wherein the average molecular weight of the xyloglucan oligomer or the functionalized xyloglucan oligomer ranges from 0.5 kDa to about 500 kDa

14. The method of any of claims 1-13, wherein the xyloglucan endotransglycosylase is present at a concentration of about 0.1 nM to about 1 mM.

15. The method of any of claims 1-14, wherein the polymeric xyloglucan or polymeric xyloglucan functionalized with a chemical group is present at about 1 mg per g of the material to about 1 g per g of the material.

16. The method of any of claims 1-15, wherein the xyloglucan oligomer or the functionalized xyloglucan oligomer is present with the polymeric xyloglucan at about 50:1 molar ratio to about 0.5:1 xyloglucan oligomer or functionalized xyloglucan oligomer to polymeric xyloglucan.

17. The method of any of claims 1-16, wherein the concentration of the polymeric xyloglucan, the polymeric xyloglucan functionalized with a chemical group, the xyloglucan oligomer, or the functionalized xyloglucan oligomer comprising a chemical group incorporated into the material is about 0.01 g to about 500 mg per g of material.

18. The method of any of claims 1-17, wherein the xyloglucan oligomer or the functionalized xyloglucan oligomer is present without the polymeric xyloglucan or the polymeric xyloglucan functionalized with a chemical group at about 1 mg per g of the material to about 1 g per g of the material.

19. The method of any of claims 1-18, wherein the chemical group is a compound of interest or a reactive group selected from the group consisting of an aldehyde group, an amino group, an aromatic group, a carboxyl group, a halogen group, a hydroxyl group, a ketone group, a nitrile group, a nitro group, a sulfhydryl group, and a sulfonate group.

## Patentansprüche

1. Verfahren zum Verbinden von Materialien, das Behandeln von zwei oder mehreren Materialien mit einer Zusammensetzung umfasst, die aus der Gruppe bestehend aus (a) einer Zusammensetzung, die eine Xyloglucanendotransglycosylase, ein polymeres Xyloglucan und ein eine chemische Gruppe umfassendes funktionalisiertes Xyloglucanoligomer umfasst; (b) einer Zusammensetzung, die eine Xyloglucanendotransglycosylase, ein mit einer chemischen Gruppe funktionalisiertes polymeres Xyloglucan, und ein eine chemische Gruppe umfassendes funktionalisiertes Xyloglucanoligomer umfasst; (c) einer Zusammensetzung, die eine Xyloglucanendotransglycosylase, ein mit einer chemischen Gruppe funktionalisiertes polymeres Xyloglucan, und ein Xyloglucanoligomer umfasst; (d) einer Zusammensetzung, die eine Xyloglucanendotransglycosylase, ein polymeres Xyloglucan und ein Xyloglucanoligomer umfasst; (e) einer Zusammensetzung, die eine Xyloglucanendotransglycosylase und ein mit einer chemischen Gruppe funktionalisiertes polymeres Xyloglucan umfasst; (f) einer Zusammensetzung, die eine Xyloglucanendotransglycosylase und ein polymeres Xyloglucan umfasst; (g) einer Zusammensetzung, die eine Xyloglucanendotransglycosylase und ein eine chemische Gruppe umfassendes funktionalisiertes Xyloglucanoligomer umfasst; und (h) einer Zusammensetzung, die eine Xyloglucanendotransglycosylase und ein Xyloglucanoligomer umfasst, ausgewählt ist, unter Bedingungen, die zu einem modifizierten Material führen, das mit einer chemischen Gruppe funktionalisiertes polymeres Xyloglucan umfasst, unter Bedingungen, die zum Verbinden zwischen den zwei oder mehreren Materialien führen, wobei das Verbinden zwischen den zwei oder mehreren Materialien über Xyloglucan oder Xyloglucanderivate erfolgt, die kovalente oder nicht kovalente Assoziationen mit den zwei oder mehreren Materialien bilden, und wobei die Xyloglucanendotransglycosidase eine Xyloglucan:Xyloglucan-Xyloglucanotransferase (EC 2.4.1.207) ist.

2. Verfahren zum Herstellen eines Verbundwerkstoffs, das Behandeln von zwei oder mehreren Materialien mit einer Zusammensetzung umfasst, die aus der Gruppe bestehend aus (a) einer Zusammensetzung, die eine Xyloglucanendotransglycosylase, ein polymeres Xyloglucan und ein eine chemische Gruppe umfassendes funktionalisiertes Xyloglucanoligomer umfasst; (b) einer Zusammensetzung, die eine Xyloglucanendotransglycosylase, ein mit einer chemischen Gruppe funktionalisiertes polymeres Xyloglucan und ein eine chemische Gruppe umfassendes funktionalisiertes Xyloglucanoligomer umfasst; (c) einer Zusammensetzung, die eine Xyloglucanendotransglycosylase, ein mit einer chemischen Gruppe funktionalisiertes polymeres Xyloglucan und ein Xyloglucanoligomer umfasst; (d) einer Zusammensetzung, die eine Xyloglucanendotransglycosylase, ein polymeres Xyloglucan und ein Xyloglucanoligomer umfasst; (e) einer Zusammensetzung, die eine Xyloglucanendotransglycosylase und ein mit einer chemischen Gruppe funktionalisiertes polymeres Xyloglucan umfasst; (f) einer Zusammensetzung, die eine Xyloglucanendotransglycosylase und ein polymeres Xyloglucan umfasst; (g) einer Zusammensetzung, die eine Xyloglucanendotransglycosylase und ein eine chemische Gruppe umfassendes funktionalisiertes Xyloglucanoligomer umfasst; und (h) einer Zusammensetzung, die eine Xyloglucanendotransglycosylase und ein Xyloglucanoligomer umfasst, ausgewählt ist, unter Bedingungen, die zu einem modifizierten Material führen, das mit einer chemischen Gruppe funktionalisiertes polymeres Xyloglucan umfasst, unter Bedingungen, die zur Assoziation zwischen den zwei oder mehreren Materialien führen, wobei das Verbinden zwischen den zwei oder mehreren Materialien über Xyloglucan oder Xyloglucanderivative erfolgt, die kovalente oder nicht kovalente Assoziationen mit den zwei oder mehr Materialien führen, und wobei die Xyloglucanendotransglycosidase eine Xyloglucan:Xyloglucan-Xyloglucanotransferase (EC 2.4.1.207) ist.

3. Verfahren nach Anspruch 1 oder 2, wobei das/die Material(ien) aus der Gruppe bestehend aus einer Cellulose, einem cellulosischen Material, einem lignocellulosischen Material, einem modifizierten cellulosischen Material, einem Cellulosederivatmaterial, einem nicht cellulosischen natürlichen Polymer, einem synthetischen Polymer und einem Mineral ausgewählt ist/sind.

4. Verfahren nach Anspruch 1 oder 2, wobei das/die Material(ien) aus der Gruppe bestehend aus Papier, Brett, Pulpe, Baumwolle, Wolle, Garn, Jute, cellulosischer Biomasse, lignocellulosischer Biomasse, Filterpapier, Celluloseacetat, Carboxymethylcellulose, Rayon, Viskose, Nitrocelluloselack, Holz, Holzfurnier, Furnierschichtholz (LVL), Holzlaminaten, Pappe, Grobspanplatte (OSB), Spanplatte und Furniersperrholz ausgewählt ist/sind.

5. Verfahren nach Ansprüchen 1-4, wobei das polymere Xyloglucan vor dem Behandeln des Materials, während des Behandelns des Materials oder nach dem Behandeln des Materials funktionalisiert wird.

6. Verfahren nach Ansprüchen 1, wobei das funktionalisierte Material anschließend durch Zugabe von Xyloglucanase, Endo-β-1-4-glucanase, Cellulase oder Kombinationen davon entfunktionalisiert wird.

7. Verfahren nach Anspruch 6, wobei das anschließend entfunktionalisierte Material durch Zugabe von mit einer chemischen Gruppe funktionalisiertem polymerem Xyloglucan oder funktionalisierten Xyloglucanoligomeren und Xyloglucanendotransglycosylase refunktionalisiert wird.

8. Verfahren nach Anspruch 1-5, wobei die verbundenen Materialien oder Verbundwerkstoffe schrittweise durch Assoziation von einem oder mehreren Materialien mit Xyloglucan und/oder Xyloglucanendotransglycosylase gefolgt von anschließender Zugabe von zusätzlichen Materialien mit oder ohne Xyloglucan und mit oder ohne Xyloglucanendotransglycosylase verbunden werden.

9. Verfahren nach Anspruch 1 oder 2, wobei die verbundenen Materialien oder Verbundwerkstoffe anschließend durch Zugabe von Xyloglucanase, Endo-β-1-4-glucanase, Cellulase oder Kombinationen davon entbunden werden.

10. Verfahren nach Ansprüchen 9, wobei die anschließend entbundenen Materialien durch Zugabe von Xyloglucan oder mit einer chemischen Gruppe funktionalisiertem polymerem Xyloglucan und Xyloglucanendotransglycosylase wieder verbunden werden.

11. Verfahren nach einem beliebigen der Ansprüche 1-10, wobei das verbundene Material oder der Verbundwerkstoff eine oder mehrere verbesserte Eigenschaften umfassen, wobei die eine oder mehrere Verbesserung(en) aus der Gruppe bestehend aus Widerstandfähigkeit gegenüber Fäule, Zersetzung, Alterung, Oxidation, Flammen- oder chemischer Widerstandfähigkeit, UV-Widerstandfähigkeit, erhöhten Farbeigenschaften, verbesserter Farbstoff- oder Farbaufnahme oder Retention, spezifischen Affinitäten, spezifischer chemischer Reaktivität, fungiziden oder fungistatischen Eigenschaften, herbiziden oder herbistatischen Eigenschaften, bakterioziden oder bakteriostatischen Eigenschaften, microbioziden oder microbiostatischen Eigenschaften, unspezifischen Affinitäten, chemischer Reaktivität oder Affinität gegenüber oder Widerstandfähigkeit gegenüber spezifischen Chemikalien, chemischen Gruppen, Toxinen, Metallen, Salzen, Ionen, Polypeptiden oder gewünschten Analyten, einschließlich biologischen, radiologischen, chemischen, etc., erhöhtem Wasserabweisungsvermögen, Wetterbeständigkeit, erhöhter Zugfestigkeit, Reißfestigkeit, veränderten oder verbesserten optischen Eigenschaften, Leitfähigkeiten, thermischen Eigenschaften, enzymatischer Aktivität, Vermischungs- oder Assoziationseigenschaften, Anti-Faltenbildung, Antipilling, Anti-Schrumpf, Oberflächeneigenschaften, Hydrophobie, Hydrophilie, Haptik, Struktur, oder Handgefühleigenschaften, magnetischen Eigenschaften, Fließeigenschaften und Zusammensetzungseigenschaften ausgewählt ist (sind).

12. Verfahren nach einem beliebigen der Ansprüche 1-11, wobei das mittlere Molekulargewicht des polymeren Xyloglucans oder des mit einer chemischen Gruppe funktionalisierten polymeren Xyloglucans im Bereich von 2 kDa bis etwa 500 kDa liegt.

13. Verfahren nach einem beliebigen der Ansprüche 1-12, wobei das mittlere Molekulargewicht des Xyloglucanoligomers oder des funktionalisierten Xyloglucanoligomers im Bereich von 0,5 kDa bis etwa 500 kDa liegt.

14. Verfahren nach einem beliebigen der Ansprüche 1-13, wobei die Xyloglucanendotransglycosylase in einer Konzentration von etwa 0,1 nM bis etwa 1 mM vorliegt.

15. Verfahren nach einem beliebigen der Ansprüche 1-14, wobei das polymere Xyloglucan oder mit einer chemischen Gruppe funktionalisierte polymere Xyloglucan zu etwa 1 mg pro g des Materials bis etwa 1 g pro g des Materials vorliegt.

16. Verfahren nach einem beliebigen der Ansprüche 1-15, wobei das Xyloglucanoligomer oder das funktionalisierte Xyloglucanoligomer mit dem polymeren Xyloglucan zu etwa 50:1 Molverhältnis bis etwa 0,5:1 Xyloglucanoligomer oder funktionalisiertes Xyloglucanoligomer zu polymerem Xyloglucan vorliegt.

17. Verfahren nach einem beliebigen der Ansprüche 1-16, wobei die Konzentration des polymeren Xyloglucans, des mit einer chemischen Gruppe funktionalisierten polymeren Xyloglucans, des Xyloglucanoligomers oder des eine chemische Gruppe umfassenden funktionalisierten Xyloglucanoligomers, die in das Material eingebracht sind, etwa 0,01 g bis etwa 500 mg pro g Material beträgt.

18. Verfahren nach einem beliebigen der Ansprüche 1-17, wobei das Xyloglucanoligomer oder das funktionalisierte Xyloglucanoligomer ohne das polymere Xyloglucan oder das mit einer chemischen Gruppe funktionalisierte polymere Xyloglucan zu etwa 1 mg pro g des Materials bis etwa 1 g pro g of des Materials vorliegt.

19. Verfahren nach einem beliebigen der Ansprüche 1-18, wobei die chemische Gruppe eine Verbindung von Interesse oder eine reaktive Gruppe ist, die aus der Gruppe bestehend aus einer Aldehydgruppe, einer Aminogruppe, einer aromatischen Gruppe, einer Carboxylgruppe, einer Halogengruppe, einer Hydroxylgruppe, einer Ketongruppe, einer Nitrilgruppe, einer Nitrogruppe, einer Sulfhydrylgruppe und einer Sulfonatgruppe ausgewählt ist.

## Revendications

1. Méthode pour lier des matériaux comprenant le traitement de deux matériaux ou plus avec une composition choisie dans le groupe constitué par (a) une composition comprenant une xyloglucane endotransglycosylase, un xyloglucane polymère, et un oligomère de xyloglucane fonctionnalisé comprenant un groupe chimique ; (b) une composition comprenant une xyloglucane endotransglycosylase, un xyloglucane polymère fonctionnalisé avec un groupe chimique, et un oligomère de xyloglucane fonctionnalisé comprenant un groupe chimique ; (c) une composition comprenant une xyloglucane endotransglycosylase, un xyloglucane polymère fonctionnalisé avec un groupe chimique, et un oligomère de xyloglucane ; (d) une composition comprenant une xyloglucane endotransglycosylase, un xyloglucane polymère, et un oligomère de xyloglucane ; (e) une composition comprenant une xyloglucane endotransglycosylase et un xyloglucane polymère fonctionnalisé avec un groupe chimique ; (f) une composition comprenant une xyloglucane endotransglycosylase et un xyloglucane polymère ; (g) une composition comprenant une xyloglucane endotransglycosylase et un oligomère de xyloglucane fonctionnalisé comprenant un groupe chimique ; et (h) une composition comprenant une xyloglucane endotransglycosylase et un oligomère de xyloglucane, dans des conditions conduisant à un matériau modifié comprenant du xyloglucane polymère fonctionnalisé avec un groupe chimique, dans des conditions conduisant à une liaison entre les deux matériaux ou plus, dans laquelle la liaison entre les deux matériaux ou plus se produit via du xyloglucane ou des dérivés de xyloglucane formant des associations covalentes ou non covalentes avec les deux matériaux ou plus, et dans laquelle la xyloglucane endotransglycosidase est une xyloglucane:xyloglucane xyloglucanotransférase (EC 2.4.1.207).

2. Méthode pour produire un matériau composite comprenant le traitement de deux matériaux ou plus avec une composition choisie dans le groupe constitué par (a) une composition comprenant une xyloglucane endotransglycosylase, un xyloglucane polymère, et un oligomère de xyloglucane fonctionnalisé comprenant un groupe chimique ; (b) une composition comprenant une xyloglucane endotransglycosylase, un xyloglucane polymère fonctionnalisé avec un groupe chimique, et un oligomère de xyloglucane fonctionnalisé comprenant un groupe chimique ; (c) une composition comprenant une xyloglucane endotransglycosylase, un xyloglucane polymère fonctionnalisé avec un groupe chimique, et un oligomère de xyloglucane ; (d) une composition comprenant une xyloglucane endotransglycosylase, un xyloglucane polymère, et un oligomère de xyloglucane ; (e) une composition comprenant une xyloglucane endotransglycosylase et un xyloglucane polymère fonctionnalisé avec un groupe chimique ; (f) une composition comprenant une xyloglucane endotransglycosylase et un xyloglucane polymère ; (g) une composition comprenant une xyloglucane endotransglycosylase et un oligomère de xyloglucane fonctionnalisé comprenant un groupe chimique ; et (h) une composition comprenant une xyloglucane endotransglycosylase et un oligomère de xyloglucane, dans des conditions conduisant à un matériau modifié comprenant du xyloglucane polymère fonctionnalisé avec un groupe chimique, dans des conditions conduisant à une association entre les deux matériaux ou plus, dans laquelle la liaison entre les deux matériaux ou plus se produit via du xyloglucane ou des dérivés de xyloglucane formant des associations covalentes ou non covalentes avec les deux matériaux ou plus, et dans laquelle la xyloglucane endotransglycosidase est une xyloglucane:xyloglucane xyloglucanotransférase (EC 2.4.1.207).

3. Méthode selon la revendication 1 ou 2, dans laquelle le ou les matériaux sont choisis dans le groupe constitué par une cellulose, un matériau cellulosique, un matériau lignocellulosique, un matériau cellulosique modifié, un matériau dérivé de cellulose, un polymère naturel non cellulosique, un polymère synthétique, et un minéral.

4. Méthode selon la revendication 1 ou 2, dans laquelle le ou les matériaux sont choisis dans le groupe constitué par le papier, le carton, la pâte à papier, le coton, la laine, le fil textile, le jute, une biomasse cellulosique, une biomasse lignocellulosique, le papier filtre, l'acétate de cellulose, la carboxyméthylcellulose, la rayonne, la viscose, le vernis nitrocellulosique, le bois, le bois en feuille mince, le bois en feuille mince stratifié (LVL), les stratifiés de bois, le carton-pâte, le carton à copeaux orientés, les panneaux de particules, et le contreplaqué.

5. Méthode selon les revendications 1 à 4, dans laquelle le xyloglucane polymère est fonctionnalisé avant le traitement du matériau, pendant le traitement du matériau, ou après le traitement du matériau.

6. Méthode selon la revendication 1, dans laquelle le matériau fonctionnalisé est subséquemment défonctionnalisé par addition de xyloglucanase, d'endo-β-1,4-glucanase, de cellulase, ou de combinaisons de celles-ci.

7. Méthode selon la revendication 6, dans laquelle le matériau subséquemment défonctionnalisé est refonctionnalisé par addition de xyloglucane polymère fonctionnalisé avec un groupe chimique ou d'oligomères de xyloglucane fonctionnalisés et de xyloglucane endotransglycosylase.

8. Méthode selon les revendications 1 à 5, dans laquelle les matériaux liés ou composites sont liés par étape, par association d'un ou plusieurs matériaux avec du xyloglucane et/ou de la xyloglucane endotransglycosylase, suivie de l'addition subséquente de matériaux additionnels avec ou sans xyloglucane et avec ou sans xyloglucane endotransglycosylase.

9. Méthode selon la revendication 1 ou 2, dans laquelle les matériaux liés ou composite sont subséquemment déliés par addition de xyloglucanase, d'endo-β-1,4-glucanase, de cellulase, ou de combinaisons de celles-ci.

10. Méthode selon la revendication 9, dans laquelle les matériaux subséquemment déliés sont reliés par addition de xyloglucane ou de xyloglucane polymère fonctionnalisé avec un groupe chimique et de xyloglucane endotransglycosylase.

11. Méthode selon l'une quelconque des revendications 1 à 10, dans laquelle le matériau lié ou composite comprend une ou plusieurs propriétés améliorées, et dans laquelle la ou les améliorations sont choisies dans le groupe constitué par la résistance à la putréfaction, à la décomposition, à la sénescence, à l'oxydation, à la flamme ou à la résistance chimique, la résistance aux UV, de meilleures propriétés de coloration, une amélioration de la rétention ou du captage des couleurs ou des colorants, des affinités spécifiques, une réactivité chimique spécifique, des propriétés fongicides ou fongistatiques, des propriétés herbicides ou herbistatiques, des propriétés bactéricides ou bactériostatiques, des propriétés microbicides ou microbiostatiques, des affinités non spécifiques, une réactivité ou affinité chimique vis-à-vis ou une résistance à des produits chimiques, groupes chimiques, toxines, métaux, sels, ions, polypeptides ou analytes souhaités, y compris biologiques, radiologiques, chimiques, etc., un meilleur caractère hydrofuge, une résistance aux intempéries, une meilleure résistance à la traction, une résistance à la déchirure, une altération ou une modification des propriétés optiques, des propriétés conductrices, des propriétés thermiques, de l'activité enzymatique, des propriétés de mélange ou d'association, des propriétés antirides, anti-placage, anti-retrait, des propriétés de surface, un caractère hydrophobe, un caractère hydrophile, le toucher, la texture, ou des propriétés de sensation à la main, des propriétés magnétiques, la rhéologie, et des propriétés de composition.

12. Méthode selon l'une quelconque des revendications 1 à 11, dans laquelle la masse moléculaire moyenne du xyloglucane polymère ou du xyloglucane polymère fonctionnalisé avec un groupe chimique est située dans la plage allant de 2 kDa à environ 500 kDa.

13. Méthode selon l'une quelconque des revendications 1 à 12, dans laquelle la masse moléculaire moyenne de l'oligomère de xyloglucane ou de l'oligomère de xyloglucane fonctionnalisé est située dans la plage allant de 0,5 kDa à environ 500 kDa.

14. Méthode selon l'une quelconque des revendications 1 à 13, dans laquelle la xyloglucane endotransglycosylase est présente à une concentration d'environ 0,1 nM à environ 1 mM.

15. Méthode selon l'une quelconque des revendications 1 à 14, dans laquelle le xyloglucane polymère ou le xyloglucane polymère fonctionnalisé avec un groupe chimique est présent à raison d'environ 1 mg par g du matériau à environ 1 g par g du matériau.

16. Méthode selon l'une quelconque des revendications 1 à 15, dans laquelle l'oligomère de xyloglucane ou l'oligomère de xyloglucane fonctionnalisé est présent avec le xyloglucane polymère en un rapport molaire de l'oligomère de xyloglucane ou de l'oligomère de xyloglucane fonctionnalisé au xyloglucane polymère d'environ 50/1 à environ 0,5/1.

17. Méthode selon l'une quelconque des revendications 1 à 16, dans laquelle la concentration du xyloglucane polymère, du xyloglucane polymère fonctionnalisé avec un groupe chimique, de l'oligomère de xyloglucane, ou de l'oligomère de xyloglucane fonctionnalisé comprenant un groupe chimique incorporé dans le matériau est d'environ 0,01 g à environ 500 mg par g de matériau.

18. Méthode selon l'une quelconque des revendications 1 à 17, dans laquelle l'oligomère de xyloglucane ou l'oligomère de xyloglucane fonctionnalisé est présent sans le xyloglucane polymère ou le xyloglucane polymère fonctionnalisé avec un groupe chimique à raison d'environ 1 mg par g du matériau à environ 1 g par g du matériau.

19. Méthode selon l'une quelconque des revendications 1 à 18, dans laquelle le groupe chimique est un composé présentant un intérêt ou un groupe réactif choisi dans le groupe constitué par un groupe aldéhyde, un groupe amino, un groupe aromatique, un groupe carboxyle, un groupe halogéno, un groupe hydroxyle, un groupe cétone, un groupe nitrile, un groupe nitro, un groupe sulfhydryle, et un groupe sulfonate.
